# EUROPEAN PATENT SPECIFICATION

(11) **EP 2 476 429 B1**
(45) Date of publication and mention of the grant of the patent: **07.03.2018**
(21) Application number: 10815369.3
(22) Date of filing: 08.09.2010
(51) Int. Cl.: A61K 38/29, A61K 33/10, A61P 19/08, A61P 19/10, A61P 43/00

(54) **PTH-CONTAINING THERAPEUTIC/PROPHYLACTIC AGENT FOR OSTEOPOROSIS, CHARACTERIZED IN THAT PTH IS ADMINISTERED ONCE A WEEK AT UNIT DOSE OF 200 UNITS**
PTH-HALTIGES THERAPIE-/PROPHYLAXE-MITTEL FÜR OSTEOPOROSE MIT EINMAL PRO WOCHE MIT EINER EINHEITENDOSIS VON 200 EINHEITEN ERFOLGENDER PTH-VERABREICHUNG
AGENT THÉRAPEUTIQUE/PROPHYLACTIQUE CONTENANT DE LA PARATHORMONE (PTH) POUR L'OSTÉOPOROSE, CARACTÉRISÉ EN CE QUE LA PTH EST ADMINISTRÉE UNE FOIS PAR SEMAINE SELON UNE DOSE UNITAIRE DE 200 UNITÉS

(30) Priority: 09.09.2009 JP 2009208039
(43) Date of publication of application: 18.07.2012
(60) Divisional application: 13181713.2
(73) Proprietor: Asahi Kasei Pharma Corporation, Tokyo 101-8101 (JP)
(72) Inventor: SHIRAE Shinichiro, Tokyo 101-8101 (JP); NAKAMURA Yasuo, Tokyo 101-8101 (JP); OYA, Yuiko, Tokyo 101-8101 (JP); NOZAKI Yoshihide, Tokyo 101-8101 (JP); KOBAYASHI Nobuyuki, Tokyo 101-8101 (JP); KURODA Tatsuhiko, Tokyo 101-8101 (JP); KATO Hiroki, Tokyo 101-8101 (JP); SERADA Masashi, Tokyo 101-8101 (JP); HORI Kazuyoshi, Tokyo 101-8101 (JP)
(74) Representative: Grünecker Patent- und Rechtsanwälte PartG mbB
(86) International application number: PCT/JP2010/065379
(87) International publication number: WO 2011/030774

(56) References cited:
- JP-A- 8 073 376
- JP-A- 2002 523 375
- JP-A- 2003 055 226
- JP-A- 2003 095 974
- JP-A- 2006 241 098
- T. FUJITA ET AL: "Effect of an Intermittent Weekly Dose of Human Parathyroid Hormone (1-34) on Osteoporosis: A Randomized Double-Masked Prospective Study Using Three Dose Levels", OSTEOPOROSIS INTERNATIONAL, vol. 9, no. 4, 1 April 1999 (1999-04-01), pages 296-306, XP055050510, ISSN: 0937-941X, DOI: 10.1007/s001980050151
- TAKAMI MIKI ET AL: "Effect and safety of intermittent weekly administration of human parathyroid hormone 1-34 in patients with primary osteoporosis evaluated by histomorphometry and microstructural analysis of iliac trabecular bone before and after 1 year of treatment", JOURNAL OF BONE AND MINERAL METABOLISM, vol. 22, no. 6, 1 November 2004 (2004-11-01), pages 569-576, XP055050478, ISSN: 0914-8779, DOI: 10.1007/s00774-004-0525-z
- DELANEY ET AL: "Strategies for the prevention and treatment of osteoporosis during early postmenopause", AMERICAN JOURNAL OF OBSTETRICS & GYNECOLOGY, MOSBY, ST LOUIS, MO, US, vol. 194, no. 2, 1 February 2006 (2006-02-01), pages S12-S23, XP005266796, ISSN: 0002-9378, DOI: 10.1016/J.AJOG.2005.08.049
- TOYONOBU UZAWA: 'Hone Calcium Taisha Ijosho no Shinki Chiryoyaku 4. Fuku Kojosen Hormone PTH (1-34) Maishu Hikatoyo Seizai' CLIN. CALCIUM vol. 17, no. 1, 2006, pages 56 - 62, XP008150788

## Description

### TECHNICAL FIELD

The present invention relates to a therapeutic or prophylactic agent for osteoporosis that contains PTH as the active ingredient. The present invention also relates to an agent to inhibit or prevent bone fractures that contains PTH as the active ingredient. In particular, the present invention relates to the above-mentioned drug, characterized in that PTH is administered once a week in a unit dose of 200 units. over a duration exceeding 48 weeks.

### BACKGROUND ART

Osteoporosis is "a disease characterized by a decrease in bone strength that poses an increased risk of bone fractures." PTH (parathyroid hormone) preparations are currently known as one agent for the treatment of osteoporosis.

PTH is a hormone that contributes to regulation of the blood calcium concentration, together with calcitonins and vitamin D. For example, PTH is also known to promote calcium absorption in the intestine by increasing active vitamin D₃ production in the kidney in vivo (Non-patent Reference 1).

Patent Reference 1 discloses a method of treating osteoporosis that increases the bone density of the cancellous bone of the osteoporosis patient without decreasing the bone density of the cortical bone by subcutaneous administration of a unit dose of 100 or 200 units of PTH to the osteoporosis patient once a week over a period of 26 weeks.

Thus, while Patent Reference 1 discloses that these treatment methods simply lead to an increase in bone density, it does not demonstrate whether or not the treatment method is capable of increasing the bone strength of the osteoporosis patient or decreasing the risk of bone fractures. PTH is also only administered alone, not in combination with calcium agents.

Non-patent Reference 1 discloses that hypercalcemia was seen in 11% of patients and that persistent hypercalcemia was observed in 3% of them when blood samples were taken 4 to 6 hours after administration of PTH (20 µg/day) to patients in a clinical trial of PTH in the treatment of osteoporosis. Furthermore, Non-patent Reference 1 also discloses that treatment was discontinued due to persistent serum calcium elevation in one of 541 patients even though the serum calcium returned to normal in virtually all patients prior to the next PTH administration.

Although Non-patent Reference 2 discloses that there was no clinical problem with the serum calcium after administration of the drug when a daily subcutaneous PTH preparation was administered together with a calcium agent, it also reports that the serum calcium was elevated after administration. Non-patent Reference 3 is the package insert of the daily subcutaneous preparation disclosed in Non-patent Reference 2. This insert reports in the disclosure of the various adverse events occurring after administration of the preparation that transient hypercalcemia was observed after administration of the preparation. Furthermore, Non-patent Reference 3 discloses that there have been adverse drug reaction reports of hypercalcemia in postmarketing surveillance of this preparation.

Thus, Non-patent References 1 to 3 disclose instances of hypercalcemia as an adverse drug reaction in the treatment of osteoporosis by PTH and the like, and the treatment methods disclosed therein cannot be called adequate in terms of safety.

Given this background, a highly safe and effective method of treating osteoporosis by PTH was desired.

### PRIOR ART REFERENCES

### PATENT REFERENCES

Patent Reference 1: JP Kokai H08-73376
Patent Reference 2: WO00/10596
Patent Reference 3: JP Kokai H05-306235
Patent Reference 4: JP Kokai S64-16799
Patent Reference 5: WO02/002136
Patent Reference 6: JP Kokai 2003-095974

### NON-PATENT REFERENCES

Non-patent Reference 1: Paul D. Miller, MD, Current Osteoporosis reports, Vol. 6, 12-16, 2008
Non-patent Reference 2: CLINICAL CALCIUM, Vol. 17, No. 1, 48-55, 2007
Non-patent Reference 3: FORTEO (registered trademark) teriparatide (rDNA origin) injection 750 mcg/3 mL, 2008
Non-patent Reference 4: ADVANCES IN ENZYMOLOGY, 32, 221-296, 1969
Non-patent Reference 5: Hoppe Seyler's Z. Physiol Chem., 355, 415, 1974
Non-patent Reference 6: J. Biol. Chem., Vol. 259, No. 5, 3320, 1984
Non-patent Reference 7: Pthobiology annual, 11, 53, 1981
Non-patent Reference 8: J. Biol. Chem., Vol. 266, 2831-2835, 1991
Non-patent Reference 9: Marcus. & Aurbach, G.D., Endocrinology 85, 801-810, 1969
Non-patent Reference 10: "2006 Osteoporosis Prevention and Treatment Guidelines" (Life Science Publishing Co.,Ltd.)
Non-patent Reference 11: M. Takai et al., Peptide Chemistry 1979, 187-192, 1980
Non-patent Reference 12: H. Orimo, et al., Diagnostic criteria for primary osteoporosis (1996 revised edition) (1997) Journal of Bone And Mineral Metabolism 14; 219-233
Non-patent Reference 13: Diagnostic criteria for primary osteoporosis and osteoporosis prevention and treatment guidelines (H. Orimo, et al., 2006 Osteoporosis prevention and treatment guidelines (2006) 34-35)
Non-patent Reference 14: Genant H.K. et al., J. Bone Miner. Res., 8, 1137-1148, 1993
Non-patent Reference 15: N. Engl. J. Med., Vol. 344, No. 19, 1434-1441, 2001
Non-patent Reference 16: Clinical Diabetes, Vo. 22, No. 1, 10-20, 2004
Non-patent Reference 17: Kawasaki Medical Journal, 36(1), 23-33, 2010
Non-patent Reference 18: Bone, Vol. 32, 86-95, 2003
Non-patent Reference 19: Reports on Dentistry, 102(11): 853-868, 2002
Non-patent Reference 20: Miner. Electrolyte Metab. 1995; 21(1-3): 201-4
Non-patent Reference 21: Nippon Seikeigeka Gakkai Zasshi 1995 Oct; 69(10): 1027-36
Non-patent Reference 22: Calcif. Tissue Int. 2000 Mar; 66(3): 229-33
Non-patent Reference 23: N. Engl. J. Med., Vol. 358, No. 12, 1302-1304, 2008
Non-patent Reference 24: N. Engl. J. Med., Vol. 357, No. 20, 2028-2039, 2007
Non-patent Reference 25: Osteoporos. Int. 2003 Jan; 14 (1) : 77-81
Non-patent Reference 26: Osteoporos. Int. 2000; 11(5): 434-42
Non-patent Reference 27: J. Bone Miner. Res. 2000 May; 15(5): 944-51
Non-patent Reference 28: J. Clin. Invest. 1998 Oct 15; 102(8): 1627-33
Non-patent Reference 29: J. Clin. Endocrinol. Metab. 2001 Feb; 86(2): 511-6
Non-patent Reference 30: J. Bone Miner. Res. 2004 May; 19(5): 745-51. Epub 2004 Jan 19
Non-patent Reference 31: J. Clin. Endocrinol. Metab. 2009 Oct; 94(10): 3772-80. Epub 2009 Jul 7
Non-patent Reference 32: Osteoporos. Int. 2007; 18: 59-68
Non-patent Reference 33: J. Bone Miner. Res., Vol. 25, No. 3, 472-481 (2010)
Non-patent Reference 34: Osteoporos. Int. 1999; 9: 296-306
Non-patent Reference 35: T. Inoue et al., Diagnostic criteria for osteoporosis--Assessment of degree of bone loss and vertebral deformity by simple vertebral roentgenogram. Ministry of Health and Welfare Life Science Research Fund Silver Science Research 1988 Report, 1990, 3.
Non-patent Reference 36: J. Bone Miner. Metab. 1998; 16(1): 27-33
Non-patent Reference 37: Proceedings of the 26th Meeting of the Japanese Society for Bone and Mineral Research, O-025, 147 (Oct. 2008)
Non-patent Reference 38: ASBMR 31st Annual Meeting, "Weekly treatment with human parathyroid hormone (1-34) for 18 months increases bone strength via the amelioration of microarchitecture, degree of mineralization, enzymatic and non-enzymatic cross-links formation in ovariectomized cynomolgus monkeys" (SA0044, FR0044).
Non-patent Reference 39: IOF World Congress on Osteoporosis & 10th European Congress on Clinical and Economic Aspects of Osteoporosis and Osteoarthritis "ONCE-WEEKLY TREATMENT WITH TERIPARATIDE FOR 18 MONTHS INCREASES BONE STRENGTH VIA THE AMELIORATE TRABECULAR ARCHITECTURE, COLLAGEN ENZYMATIC AND NON-ENZYMATIC CROSS-LINK FORMATION IN OVARIECTOMIZED CYNOMOLGUS MONKEYS"

### SUMMARY OF THE INVENTION

### PROBLEMS TO BE SOLVED BY THE INVENTION

The present invention provides a highly safe and effective method for treating or preventing osteoporosis by PTH. Furthermore, the present invention provides a highly safe method for inhibiting or preventing bone fractures by PTH.

### MEANS TO SOLVE THE PROBLEMS

As a result of in-depth research and development intended to solve the above problems, the present inventors discovered a highly safe and effective method of treating or preventing osteoporosis, surprisingly enough, by limiting the PTH dose and administration interval. They also discovered a highly safe method of inhibiting/preventing bone fractures by specifying the PTH dose and administration interval. Furthermore, they also discovered that these methods are especially effective in high-risk patients.

Specifically, the present invention relates to the following.

An agent for the treatment or prevention of osteoporosis that contains PTH as the active ingredient, characterized in that PTH is administered once a week in a unit dose of 200 units over a duration exceeding 48 weeks, the agent for the treatment or prevention of osteoporosis being used to treat osteoporosis patients who satisfy all of the following conditions (1) through (3):
(1) Age 65 years or older
(2) Prevalent bone fractures
(3) Bone density less than 80% of the young adult mean and/or degree of bone atrophy of I or higher.

### EFFECT OF THE INVENTION

The osteoporosis therapeutic agent of the present invention is highly safe and has excellent efficacy. The agent for the inhibition or prevention of bone fractures of the present invention is also highly safe and useful.

### BRIEF DESCRIPTION OF THE DRAWINGS

Figure 1 is a graph showing the changes in the serum calcium concentration by administration group (high-risk patients, low-risk patients);

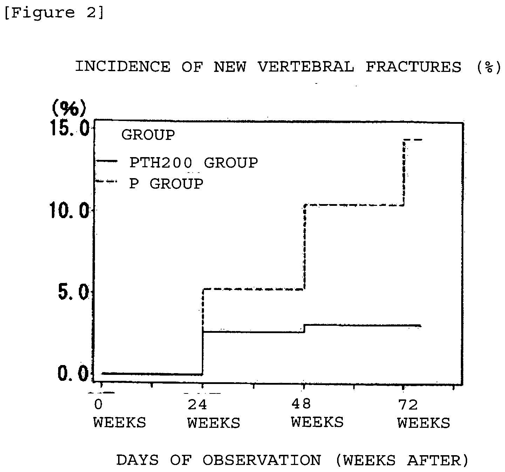

Figure 2 shows the effects of administration of the test drug on the changes over time in the incidence of new vertebral fractures. The test drug group is listed as the "PTH200 group" and the control group as the "P group;"

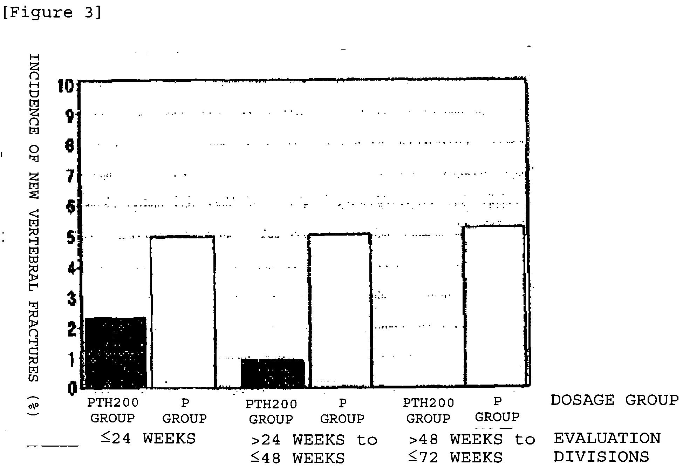

Figure 3 shows the effects of administration of the test drug on the changes over time in the incidence of new vertebral fractures. The test drug group is listed as the "PTH200 group" and the control group as the "P group;"

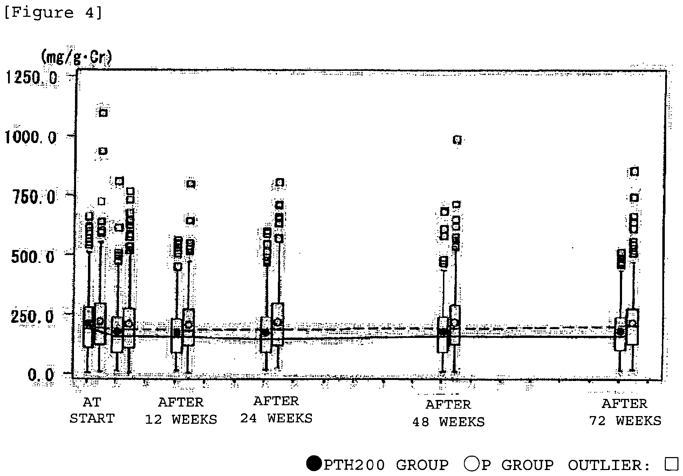

Figure 4 shows the results on the changes in the urinary calcium value when the test drug ("PTH200 group") or control ("P group") was administered to patients once a week for 72 weeks. The ratio of the urinary calcium value/urinary creatine value was compared before beginning administration and in the weeks of observation. The urinary calcium was measured at the start, after 12 weeks, after 24 weeks, after 48 weeks, and after 72 weeks. The standard concomitant drug (610 mg of calcium, 400 IU of vitamin D₃, and 30 mg of magnesium) was taken once a day after dinner from the time of consent to the end of the trial; and
Figure 5 shows the results on the changes in the corrected serum calcium value when the test drug ("PTH200 group") or control ("P group") was administered to patients once a week for 72 weeks. The serum calcium was measured at the start, after 12 weeks, after 24 weeks, after 48 weeks, and after 72 weeks. Serum calcium standard value: 8.4 to 10.4 mg/dL. The standard concomitant drug (610 mg of calcium, 400 IU of vitamin D₃, and 30 mg of magnesium) was taken once a day after dinner from the time of consent to the end of the trial.

### MODE FOR CARRYING OUT THE INVENTION

The present invention is explained concretely below.

The present invention provides a method for the treatment or prevention of osteoporosis or a method for the inhibition or prevention of bone fractures using PTH, characterized in that PTH is administered once a week in a unit dose of 200 units over a duration exceeding 48 weeks ("once a week" is also sometimes referred to as "every week" hereinafter). The present invention also provides an agent for the treatment or prevention of osteoporosis or an agent for the inhibition or prevention of bone fractures having PTH as the active ingredient, characterized in that PTH is administered every week in a unit dose of 200 units over a duration exceeding 48 weeks. Furthermore, the present invention provides the use of PTH to produce the above agent for the treatment or prevention of osteoporosis or the above agent for the inhibition or prevention of bone fractures.

### I. Active ingredient

The PTH that is the active ingredient of the present invention (sometimes referred to simply hereinafter as "PTH") includes human PTH (1-84) which is human parathyroid hormone and peptides of a molecular weight of about 4,000 to 10,000 having activity equivalent or similar to human PTH (1-84).

PTH includes all of natural PTH, PTH produced by genetic engineering techniques, and PTH synthesized by chemical synthesis methods. PTH can be produced by genetic engineering techniques that are themselves known (Non-patent Reference 8). Alternatively, PTH can be synthesized by peptide synthesis methods that are themselves known (Non-patent Reference 11). For example, it can be synthesized by a solid phase method that extends a peptide chain from the C end on an insoluble polymer carrier (Non-patent Reference 4). Furthermore, the origin of the PTH of the present invention is not limited to human and may include the rat, cow, pig, and the like.

In this specification, human PTH (n-m) means a peptide shown by a partial amino acid sequence consisting of from number n to number m in the amino acid sequence of human PTH (1-84). For example, human PTH (1-34) means a peptide shown by a partial amino acid sequence consisting of from 1 to 34 in the amino acid sequence of human PTH (1-84).

The PTH that is the active ingredient of the present invention may also be a salt formed with one, two, or more kinds of volatile organic acids. Examples of volatile organic acids include trifluoroacetic acid, formic acid, acetic acid, and the like. Acetic acid can be given as a preferred example. The ratio of the two when free PTH and a volatile organic acid form a salt is not particularly restricted as long as a salt is formed. For example, since human PTH (1-34) has nine basic amino acid residues and four acidic amino acid residues in its molecule, taking into consideration salt formation in these molecules, the basic amino acid 5 residue can be made into a chemical equivalent of acetic acid. For example, if an acetic acid content represented by the acetic acid weight × 100(%)/peptide weight of human PTH (1-34) is used as the amount of acetic acid, as one theory, the chemical equivalent of acetic acid versus free human PTH (1-34) becomes approximately 7.3% (wt%). In this specification, free human PTH (1-34) sometimes is also called teriparatide, and the acetate of teriparatide is sometimes also called teriparatide acetate. The acetic acid content in teriparatide acetate is not particularly restricted as long the teriparatide and acetic acid form a salt. For example, it may be 7.3%, which is the above theoretical chemical equivalent, or higher, or it may be 0 to 1%. More concrete examples of the acetic acid content in teriparatide are 1 to 7%, preferably 2 to 6%. These salts can be produced by methods that are themselves known (Patent References 4 and 5).

Examples of PTH include human PTH (1-84), human PTH (1-34), human PTH (1-38), hPTH (Non-patent Reference 5), human PTH (1-34) NH₂, [Nle^{8, 18}] human PTH (1-34), [Nle^{8, 18}, Tyr³⁴] human PTH (1-34), [Nle^{8, 18}] human PTH (1-34) NH₂, [Nle^{8, 18}, Tyr³⁴] human PTH (1-34) NH₂, murine PTH (1-84), murine PTH (1-34), bovine PTH (1-84), bovine PTH (1-34), bovine PTH (1-34) NH₂, and the like. Examples of preferred PTH are human PTH (1-84), human PTH (1-38), human PTH (1-34), and human PTH (1-34) NH₂ (Patent Reference 3, etc.). Human PTH (1-34) is an example of especially preferred PTH. Human PTH (1-34) obtained by chemical synthesis is an example of even more preferred PTH, and teriparatide acetate (Working Example 1) is an example of most preferable PTH.

### II. Combined use with other drugs

In the results of a double-blind comparative clinical trial of PTH used in combination with calcium agents conducted by the present inventors with the occurrence of bone fractures as the primary endpoint, the effect appeared early (after 24 or 26 weeks) and hypercalcemia was not confirmed as an adverse event (Working Examples 1 and 2). Therefore, combined use with other drugs is one characteristic of the osteoporosis therapeutic agent or agent for the inhibition/prevention of bone fractures of the present invention. Combined use with other drugs means that the osteoporosis therapeutic agent or agent for the inhibition/prevention of bone fractures of the present invention is used in combination with another drug (other drugs).

Calcium can be given as a preferred example of another drug in the present invention. However, saying combined use with other drugs in the present invention does not exclude further combined use with drugs other than these. Therefore, preferred examples of combined use with calcium include:
combined use with calcium alone and
combined use with calcium and vitamin D (including derivatives thereof) and/or magnesium alone.
Calcium agents can be given as examples of concrete embodiments of other drugs, and preferred examples include:
   (1) A calcium agent containing calcium as the active pharmaceutical ingredient and
   (2) A calcium agent containing calcium, vitamin D (including derivatives thereof), and magnesium as active pharmaceutical ingredients.

The mode of the above combined use of the osteoporosis therapeutic agent or agent for the inhibition/prevention of bone fractures of the present invention and other drugs (frequency of administration, route of administration, site of administration, dose, and the like) is not particularly restricted and can be decided as is appropriate by doctor's prescription and the like in accordance with the patient.

For example, when a calcium agent is used in combination as the above other drug, this calcium agent may be administered simultaneously with the osteoporosis therapeutic agent or agent for the inhibition/prevention of bone fractures of the present invention having PTH as the active ingredient (in other words, once a week) or may be administered more frequently from once to several times a day. Therefore, the above other drug may be made into a combination preparation in combination with the osteoporosis therapeutic/prophylactic or agent for the inhibition/prevention of bone fractures of the present invention or may be a separate preparation from the osteoporosis therapeutic/prophylactic or agent for the inhibition/prevention of bone fractures of the present invention. Examples of such calcium agents include "New Calcichew (trademark) D₃" (sold by: Daiichi Sankyo Health Care, manufactured and sold by: Nitto Pharmaceutical Industries, Ltd.).

Other drugs can also be administered by the same or a different route of administration together or sequentially (that is, at separate times) with the osteoporosis therapeutic/prophylactic agent or agent to inhibit/prevent bone fractures of the present invention. The form of the other drugs is therefore not particularly restricted. Examples include tablets, capsules, granules, and the like. When the other drug is a calcium agent, it is preferably a calcium agent containing a unit dose of 100 to 400 (preferably 150 to 350) mg as calcium. If a calcium agent containing a unit dose of 100 to 400 mg as calcium is administered, for example, at a dosage of two tablets per day according to the working example of the present invention, 200 to 800 mg per day will be administered as calcium. Possibilities, however, are not limited to this.

Concrete examples of the above other drugs, in the case of calcium agents, for example, include known drugs having as the active ingredient precipitated calcium carbonate, calcium lactate, calcium carbonate, calcium chloride, calcium gluconate, calcium aspartate, calcium phosphate, calcium hydrogen phosphate, calcium citrate, and the like. Drugs that contain precipitated calcium carbonate are preferred. Furthermore, this drug may contain as is appropriate excipients, binders, disintegrating agents, glossing agents, antacids, and the like.

Vomiting, nausea, queasiness, dull feeling in the stomach, gastric discomfort, heartburn, and other such gastrointestinal symptoms are known to be observed transiently in a certain percentage of patients administered PTH (Patent Reference 6).

As a result of testing the times of administration and efficacy of various antiemetics to counteract the transient nausea and vomiting associated with administration of the test drug, the present inventors confirmed that Primperan (generic name of the active ingredient: metoclopramide), Nauzelin (generic name of the active ingredient: domperidone), Gaster D (generic name of the active ingredient: famotidine), Gasmotin (generic name of the active ingredient: mosapride citrate), Takepron OD (generic name of the active ingredient: lansoprazole) and rokushingan are effective against the nausea and vomiting associated with PTH administration (Working Example 2). Therefore, these antiemetics are preferred as other drugs, and Nauzelin (generic name of the active ingredient: domperidone), Gasmotin (generic name of the active ingredient: mosapride citrate) and/or rokushingan can be given as preferred examples. The dosage and administration of these antiemetics can be established as is appropriate by the physician or the like in according with the patient's symptoms and other such factors.

### III. Administration period

The administration period of the osteoporosis therapeutic/prophylactic agent or agent to inhibit/prevent bone fractures of the present invention is over a duration exceeding 48 weeks. The present inventors conducted a double-blind comparative clinical trial with the occurrence of bone fractures as the primary endpoint using administration periods of 156 or 72 weeks. It was possible to confirm a significant inhibitory effect on bone fractures by such administration in this trial, and the effect appeared early (after 24 or 26 weeks) (Working Examples 1 and 2). Furthermore, no new vertebral fractures were found when administration went beyond 48 weeks (Working Example 2). Therefore, examples of administration periods include 48 weeks or longer, 52 weeks or longer, 72 weeks or longer, and 78 weeks or longer. Seventy-eight weeks or longer is most preferred. Hypercalcemia was not confirmed as an adverse event in this trial (Working Example 1).

### IV. Dose

As a result of a double-blind comparative clinical trial using a unit dose of 100 or 200 units of PTH, the present inventors found a significant inhibitory effect on bone fractures by this administration, that the effect appeared early (after 24 or 26 weeks), and that no hypercalcemia could be confirmed as an adverse event (Working Examples 1 and 2).

Therefore, one of the characteristics of the present invention is that the unit dose is 200 units. Here, one unit of PTH can be measured by activity measurement methods that are themselves known (Non-patent Reference 9). The unit dose is 200 units.

### V. Administration interval

As a result of a double-blind comparative clinical trial in which PTH was administered once a week, the present inventors found a significant inhibitory effect on bone fractures by this administration, that the effect appeared early (after 24 or 26 weeks), and that no hypercalcemia could be confirmed as an adverse event (Working Examples 1 and 2). An administration interval of once a week is therefore one characteristic of the present invention.

### VI. Route of administration

The osteoporosis therapeutic/prophylactic agent and agent to inhibit/prevent bone fractures of the present invention can be administered by an administration route that matches the form of the preparation. For example, when the osteoporosis therapeutic or prophylactic or agent to inhibit or prevent bone fractures of the present invention is an injection, it can be administered intravenously, intra-arterially, subcutaneously, intramuscularly, and the like. The present inventors established the subcutaneous injection of PTH to have excellent efficacy and safety (Working Examples 1 and 2). Subcutaneous administration can therefore be given as a preferred example of the route of administration in the present invention.

### VII. Target diseases

Osteoporosis in the present invention is not particularly restricted and includes both primary osteoporosis and secondary osteoporosis. Examples of primary osteoporosis include involutional osteoporosis (postmenopausal osteoporosis and senile osteoporosis) and idiopathic osteoporosis (postpregnancy osteoporosis, juvenile osteoporosis, and the like). Secondary osteoporosis is osteoporosis triggered by a specific disease, specific drug, or other such cause. Examples of causes include specific drugs, rheumatoid arthritis, diabetes, hyperthyroidism, sexual dysfunction, immobility, nutrition, congenital diseases, and the like. Glucocorticoid are an example of specific drugs. Osteoporosis that poses a high risk of bone fractures is a preferred example of osteoporosis in the present invention. The indication of the present invention for osteoporosis posing a high risk of bone fractures means that the present invention is indicated for the following high-risk patients.

The present inventors confirmed the efficacy and safety of the present invention in a clinical trial conducted in patients with primary osteoporosis(Working Examples 1 and 2). Primary osteoporosis can therefore be given as a preferred example of osteoporosis in the present invention, and involutional osteoporosis can be given as an especially preferred example.

The present inventors confirmed the effect of the present invention in a clinical trial conducted in primary osteoporosis patients who took glucocorticoid that trigger secondary osteoporosis (Working Example 2). Primary osteoporosis patients who take glucocorticoid that trigger secondary osteoporosis can therefore be given as a preferred example of primary osteoporosis patients in the present invention.

The present inventors confirmed the effect of the present invention in a clinical trial conducted in primary osteoporosis patients having complications (diabetes, hypertension, or hyperlipidemia) (Working Example 2). Osteoporosis patients having at least one complication among diabetes, hypertension, and hyperlipidemia can therefore be given as a preferred example of osteoporosis patients in the present invention, and primary osteoporosis patients having at least one complication among diabetes, hypertension, and hyperlipidemia can be given as a more preferred example.

Diabetes is known to very likely be an osteoporotic bone fracture risk factor (Non-patent Reference 16).

The following reports are found involving animal studies of the relationship between diabetic osteoporosis and PTH.
1) Administration of PTH to streptozotocin-treated rats that exhibited diabetic bone loss is reported to increase "bone volume," "trabecular thickness," "osteoid surface," "mineralized surface," "mineral apposition rate," and "bone formation rate" in the cancellous envelope and to increase "osteoid surface," "mineralized surface," "mineral apposition rate," and "cortical bone thickness" in the endocortical envelope (Non-patent Reference 21). However, unlike rats with bone loss due to other causes, no remarkable decrease in eroded surface is seen in these rats.
2) Recovery of cancellous bone volume and turnover is reported as a result of administration of PTH over eight weeks to streptozotocin-treated rats (Non-patent Reference 22).
3) The response to hPTH (1-34) is reported to drop (deterioration of the effectiveness of PTH) upon exposure to high-concentration glucose in experiments in cultured cells (Non-patent Reference 20).

The present inventors found that many physicians and the like expect PTH administration to be effective in human patients with diabetic osteoporosis (e.g.: http://www.richbone.com/kotsusoshosho/basic_shindan/tonyo.htm) but on the other hand discovered no papers that prove this effect.

It is therefore important that these trials proved that the osteoporosis therapeutic agent and agent to inhibit/prevent bone fractures of the present invention reduces the risk of vertebral fractures in patients having primary osteoporosis complicated by diabetes.

The bone fractures in the present invention are not particularly restricted. They include both vertebral and non-vertebral fractures (Working Example 1) and pathological fractures caused by osteoporosis, osteogenesis imperfecta, bone tumors, and the like and traumatic fractures caused by traffic accidents, blows, and the like. Preferred examples are application to fractures caused by osteoporosis, more preferably vertebral fractures caused by osteoporosis. The site of the fracture is also not particularly restricted. Typical examples also include vertebral compression fractures, fractures of the neck of the femur, intertrochanteric fractures of the femur, fractures of the shaft of the femur, fractures of the neck of the humerus, and fractures of the distal radius. Vertebral compression fractures can be given as a particular example.

The number of bone fractures is not particularly restricted in the present invention and includes both single fractures and multiple fractures. The term single fracture means a disease state in which the bone is broken or cracked at only one location. Multiple fractures means a disease 0state in which the bone is broken or cracked in two or more locations. The number of fractures in multiple fractures is not particularly restricted, but application to 2 to 4 fractures is preferred.

The vertebral fractures in the present invention also include both new fractures and worsening fractures. For example, the extent of deformity can be classified by grade by examining the shape of the entire vertebra and is generally classified as grade 0 (normal), grade 1 (decrease of approximately 20 to 25% in vertebral height and decrease of 10 to 20% in vertebral area), grade 2 (decrease of approximately 25 to 40% in vertebral height and decrease of 20 to 40% in vertebral area), and grade 3 (decrease of approximately 40% or more in vertebral height and decrease of 40% or more in vertebral area). New and worsening [fractures] can be classified by the grade increase pattern according to the criteria of Genant. Concretely speaking, a fracture is diagnosed as a new fracture when a change is found from grade 0 to grade 1, 2, or 3. It can be regarded as a worsening fracture when a change is found from grade 1 to grade 2 or 3 or from grade 2 to grade 3. The vertebral height was measured according to the method of Inoue et al. (Non-patent reference 35) and the method of Hayashi et al. (Non-patent Reference 36) to accurately assess the changes in grade.

In a clinical trial conducted in patients having prevalent fractures, the present inventors proved the inhibitory effect of the present invention on worsening fractures (Working Example 2). Patients having prevalent bone fractures can therefore be given as preferred examples, and patients having prevalent bone fractures and the possibility of worsening fractures can be given as even more preferred examples as osteoporosis patients in the present invention.

Many aspects of the mechanism of the bone strength-enhancing effect of PTH remain unclear. Bone strength reflects not only bone density but also bone quality. This means that not only bone density but also bone microstructures, mineralization, and other such bone quality factors determine bone strength (Non-patent Reference 17). The present inventors believe that bone quality may affect not only bone strength but also the risk of developing diseases other than osteoporosis and the curative results of complications thereof. The possibility has been suggested that the osteoporosis therapeutic/prophylactic agent and agent to inhibit/prevent bone fractures of the present invention is superior to existing therapeutic agents (Patent Reference 2) in this regard.

Patent Reference 2 discloses that not only the bone mineral content (BMC) and bone mineral density (BMD) but also the bone area of the lumbar spine, femur, and the like were increased as a result of administration of rhPTH (1-34) to osteoporosis patients. An increase in bone area means thickening along the outside of the bone.

However, the cortical bone thickness increased on the inside of the bone rather than the outside as a result of administering the osteoporosis therapeutic/prophylactic agent and agent to inhibit/prevent bone fractures of the present invention to osteoporosis patients. In other words, virtually no change was found in the thickness of the bone as a whole. This mechanism is believed to have the following clinical significance.

### (1) No joint damage due to thickening of the long bones

The femur, which is one of the long bones (long bones that construct the four limbs), has its epiphysis in contact with the joint cartilage and forms the knee joint together with the synovium and meniscus. The contact surface is called the joint surface and is covered with cartilage several millimeters thick. Osteoarthritis of the knee can be given as an example of a disease that causes knee joint pain.

On the other hand, Forteo (PTH for daily administration) is known to have a stronger bone strengthening effect than Fosamax in patients having prednisone-induced osteoporosis complicated by arthralgia (Non-patent References 23 and 24).

However, this Forteo administration is a conventional treatment method substantially equivalent to the PTH administration described in Patent Reference 2, and conventional methods are treatment methods that thicken the outside of the bone, as was mentioned above. Thickening of the outside of the femur means an increase in the area of the joint surface. Since the number of cartilage cells does not increase in proportion to the thickening of the bone, the thickening of the outside of the femur induced by this conventional treatment method can promote destruction of the joint via damage to the cartilage cells triggered or exacerbated by the increase in joint surface.

However, thickening on the inside of the femur as in the present invention stabilizes the cartilage without increasing the joint area. The inventors believe that as a result there is a possibility that it does not increase the burden on the cartilage and in essence does not promote joint destruction. This suggests the possibility that osteoporosis treatment by this drug is a superior treatment for the joints in comparison to osteoporosis treatment by the above conventional method.

### (2) Osteoarthritis of the spine is not exacerbated or induced by thickening of the vertebrae

The normal vertebral bone mass decreases and the vertebrae become unstable for some reason, such as aging. Destabilization begins with deformation of the end plates. Destabilization of the vertebrae specifically means thinning of the end plates and widening of the end plate holes (Haversian canals). As this destabilization advances, penetration of the intervertebral disks into the end plate holes and narrowing of the intervertebral disks are seen. As the symptoms advance further, osteophytes form due to impact between the vertebrae. Such degeneration of the spine is a condition called osteoarthritis of the spine. When osteoarthritis of the spine develops, it produces pain caused by penetration of the intervertebral disks due to intervertebral stabilization, pain due to swelling of the surrounding muscle, and the like.

However, when the outside of the bone is thickened by daily administration of PTH as described in Patent Reference 2, there is a possibility that an adequate inhibitory effect on widening of the end plate holes will not be seen. There is also a possibility that an increase in the contact area between the vertebrae and the intervertebral disks will shrink the distance between the vertebrae and promote destabilization of the vertebrae, resulting in an increased risk of the development or exacerbation of osteoarthritis of the spine.

On the other hand, since the osteoporosis therapeutic agent and agent to inhibit/prevent bone fractures of the present invention increase the cortical bone thickness on the inside of the bone rather than the outside, there is a possibility that widening of the end plate holes and penetration of the intervertebral disks into the end plate holes can be adequately inhibited.

### (3) Does not exacerbate or promote the development of osteoarthritis of the hip or osteoarthritis of the jaw

Osteoarthritis of the hip is a condition in which the acetabulum that forms the hip joint and the joint cartilage of the contact surface with the femoral head become worn, degenerate, and undergo irreversible changes, caused by poor blood flow to the joint and extreme weight and overuse. The femoral cortical bone area of patients with osteoarthritis of the hip is significantly larger than that of normal persons (Non-patent Reference 18). An increase in the femoral cortical bone area means thickening of the outside of the femur. There is consequently a possibility that this will contribute to the development or exacerbate osteoarthritis of the hip. When the inside of the femur is thickened as in the present invention, there is a possibility that the risk of development or exacerbation of osteoarthritis of the hip will not be increased since the outside of the femur is not thickened. Deformity of the jaw joint is the main sign of osteoarthritis of the jaw, but thickening of the cortical bone is one of the clinical findings (Non-patent Reference 19). Therefore, there is a possibility that further thickening of the outside of the cortical bone will exacerbate the symptoms or induce onset. When the inside of the bone is thickened as in the present invention, there is assumed to be a possibility that such risk of development or exacerbation of osteoarthritis of the jaw will not be increased.

To bring together (1) to (3) above, osteoporosis patients having at least one complication consisting of arthralgia, osteoarthritis of the spine, metatypical low back pain, osteoarthritis of the hip, and osteoarthritis of the jaw (preferably primary osteoporosis patients among them) can be given as preferred examples of patients for whom the osteoporosis therapeutic/prophylactic and agent to inhibit/prevent bone fractures of the present invention is indicated.

The present inventors evaluated the effects of other osteoporosis drugs taken within one year on the efficacy of this drug. As a result, it became clear that the efficacy of the test drug is higher in primary osteoporosis patients who have a history of taking other osteoporosis drugs than in those who do not (Working Example 2). Therefore, osteoporosis patients who have a history of taking other osteoporosis therapeutic agents can be given as a preferred example of osteoporosis patients in the present invention, and application to primary osteoporosis patients who have a history of taking other osteoporosis therapeutic agents is even more preferred.

Examples of other osteoporosis therapeutic agents include calcium L-aspartate, Alfacalcidol, raloxifene hydrochloride, elcatonin, menatetrenone, and calcium lactate. Preferred examples are calcium L-aspartate, Alfacalcidol, and elcatonin. The other osteoporosis therapeutic agents may have been given individually or in combination.

The osteoporosis therapeutic agent and agent to inhibit/prevent bone fractures of the present invention is preferably administered for 48 to 72 weeks or longer to osteoporosis patients who have a history of taking other osteoporosis therapeutic agents. Administration for 24 weeks or longer is especially preferred for patients having a high risk of lumbar vertebral fractures, and administration for 72 weeks or longer is especially preferred for patients having a high risk of fractures of the neck of the femur or the proximal femur.

The incidence of osteoporosis and renal dysfunction increases with age. A large-scale epidemiological research report states that 85% of female osteoporosis patients have mild to moderate renal dysfunction (Non-patent Reference 32). It is therefore important to provide a drug that is safe and effective for osteoporosis patients who have renal dysfunction.

The present inventors have demonstrated that the osteoporosis therapeutic/prophylactic agent and agent to inhibit/prevent bone fractures of the present invention is effective in osteoporosis patients having normal renal function, osteoporosis patients having mild renal dysfunction, and osteoporosis patients having moderate renal dysfunction (Working Example 2). Moreover, they clarified that the osteoporosis therapeutic/prophylactic agent and agent to inhibit/prevent bone fractures of the present invention has equal safety in terms of serum calcium in all of these groups.

Normal renal function, dysfunction, and the degree of dysfunction can be classified based on the creatinine clearance. Concretely speaking, creatinine clearance of 80 mL/min or higher can be assessed as normal renal function, from 50 to less than 80 mL/min as mild renal dysfunction, and from 30 to less than 50 mL/min as moderate renal dysfunction.

The upper limit of normal of the serum calcium is generally 10.6 mg/mL. Above this, 11.0 mg/mL can be called slightly high. In conventional daily administration of PTH, 11.76% of osteoporosis patients having moderate renal dysfunction were found to have serum calcium exceeding 11.0 mg/mL, which is slightly high, after administration (Non-patent Reference 32). However, not even one patient with serum calcium exceeding 11.0 mg/mL could be found at any time of testing from the start to end of administration when the osteoporosis therapeutic/prophylactic agent and agent to inhibit/prevent bone fractures of the present invention was administered to osteoporosis patients having moderate renal dysfunction in the present invention (Working Example 2). In other words, the osteoporosis therapeutic/prophylactic and agent to inhibit/prevent bone fractures of the present invention appears to be superior not only in terms of efficacy but also safety. Osteoporosis patients having mild renal dysfunction and/or osteoporosis patients having moderate renal dysfunction can therefore be given as examples of patients for whom the present invention is indicated. More preferred examples include primary osteoporosis patients having mild renal dysfunction and/or primary osteoporosis patients having moderate renal dysfunction.

The race, age, gender, height, weight, and the like of the subjects for whom the drug administration or treatment method of the present invention is indicated are not particularly restricted, but osteoporosis patients are examples of these subjects. It is desirable to apply the method of the present invention or administer the osteoporosis therapeutic agent or agent to inhibit or prevent bone fractures of the present invention to osteoporosis patients having many risk factors for bone fractures in osteoporosis. Risk factors for bone fractures in osteoporosis include age, gender, low bone density, prevalent bone fractures, smoking, alcohol consumption, steroid use, family history of bone fractures, exercise, factors related to falls, bone metabolism markers, weight, calcium intake, and the like (Non-patent Reference 10). However, osteoporosis patients (or subjects) who satisfy all of the following conditions (1) through (3) are defined as "high-risk patients" in the present invention.
(1) Age 65 years or older
(2) prevalent bone fractures
(3) Bone density less than 80% of the young adult mean and/or degree of bone atrophy of I or higher.

Here, the bone density typically means the bone mineral density of the lumbar spine. However, when it is difficult to evaluate the bone mineral density of the lumbar spine, the bone density can be represented by the bone mineral density of the radius, second metacarpal, neck of the femur, or calcaneus. The young adult mean means the average bone density of 20 to 44-year olds. The bone density can be measured by methods that are themselves known, for example, dual-energy x-ray absorptiometry, photodensitometry, photon absorptiometry, quantitative CT scan, quantitative ultrasonography, and the like. The degree of bone atrophy means the degree of bone weight loss on an x-ray in the present invention. The degree of bone atrophy is classified as no bone atrophy, bone atrophy grade I, bone atrophy grade II, and bone atrophy grade III. No bone atrophy in this scale means a normal condition, that is, a condition in which the trabecular structure cannot be recognized because the latitudinal and longitudinal trabeculae are dense. Grade I bone atrophy means that the longitudinal trabeculae are prominent. Typically, the longitudinal trabeculae are thin and visible but still arranged densely. It also means a state in which the vertebral end plates are prominent. Grade II bone atrophy in this scale means a state in which the longitudinal trabeculae have become rough, appear thick, and are roughly arranged, and the vertebral end plates are also lighter. Grade III bone atrophy in this scale means that the longitudinal trabeculae have become indistinct, the vertebral shadows overall appear blurry, and the difference from the shadows of the intervertebral disks is reduced (Osteoporosis Treatment, 5/3, July 2006 number, "Diagnosis of osteoporosis by simple x-ray"). The degree of bone atrophy can be assessed, for example, from lateral x-rays of the lumbar spine. The number of vertebral fractures in the present invention can be measured easily, for example, by the method of Genant et al. (Non-patent Reference 14). Bone fractures at locations other than the vertebrae can be confirmed easily, for example, using roentgen film.

It is especially preferable in the present invention to apply the method of the present invention or to administer the osteoporosis therapeutic agent, prophylactic agent, or the agent to inhibit or prevent bone fractures of the present invention to high-risk patients in particular (Working Example 1) .

On the other hand, it is generally preferable to avoid applying the method of the present invention or administering the osteoporosis therapeutic agent, prophylactic agent, or the agent to inhibit or prevent bone fractures of the present invention to patients (subjects) to whom any of the following (1) to (6) pertain.
(1) Patients with bronchial asthma or a tendency to rash (erythema, urticaria, and the like), and other such hypersensitivity responses
(2) Patients with hypercalcemia
(3) Women who are pregnant or might become pregnant
(4) Patients with hypothyroidism or hyperparathyroidism
(5) Patients who have exhibited drug hypersensitivity in the past
(6) Patients having heart disease, liver disease, kidney disease, or other such serious complications

Therefore, the above high-risk patients and osteoporosis patients to whom none of the above (1) through (6) pertain are the preferred subjects in the present invention.

### VIII. Preparation

The osteoporosis therapeutic/prophylactic agent and agent to inhibit/prevent bone fractures of the present invention (sometimes referred to simply hereinafter as "this drug") can take on the form of various preparations. This drug is generally made into an injection of PTH alone or together with an ordinary pharmacologically acceptable carrier. An injection is the preferred form of this drug.

For example, when this drug is an injection, it can be prepared by dissolving PTH in a suitable solvent (sterilized water, buffer, physiological saline solution, or the like), sterilizing the solution by filtration by a filter or the like and/or another appropriate method, and dispensing it into sterile containers. It is preferable in this case to add any necessary additives (for example, excipients, stabilizers, dissolution auxiliaries, antioxidants, analgesics, isotonizing agents, pH regulators, preservatives, and the like) together with the PTH. Examples of such additives include sugars, amino acids, sodium chloride, and the like. When sugars are used as additives, it is preferable to add one weight or more (preferably 50 to 1000 weights) of mannitol, glucose, sorbitol, inositol, sucrose, maltose, lactose, or trehalose as sugars per weight of PTH. When sugars and sodium chloride are used as additives, it is preferable to add 1/1000 to 1/5 weight (preferably 1/100 to 1/10 weight) of sodium chloride per weight of sugars.

For example, when this drug is an injection, this drug may be solidified by freeze drying or another such means (freeze-dried preparation or the like) and may be dissolved in an appropriate solvent at the time of use. Alternatively, when this drug is an injection, this drug may be a liquid dissolved in advance.

This drug is preferably housed in a package that states that a unit dose of 200 units of human PTH (1-34) should be administered weekly as an osteoporosis therapeutic agent and agent to inhibit/prevent bone fractures or housed in a package together with a package insert that contains this statement.

Furthermore, the utility of the present invention can be confirmed easily by statistical treatment of the results of the clinical trials that appear in the working examples by the usual methods. The present invention is explained more concretely below through working examples, but the scope of the present invention is not limited to the following working examples.

### WORKING EXAMPLES

### (Working Example 1)

Male and female patients diagnosed with primary osteoporosis (Non-patent Reference 12) were subcutaneously administered 5 or 100 units of teriparatide acetate, prepared by the method of Takai (Patent References 4 and 5 and Non-patent Reference 11), once a week (designated respectively as the 5 unit group or 100 unit group). Furthermore, the activity of the teriparatide acetate was measured according to the paper of Marcus et al. (Non-patent Reference 9).

In the 5 and 100 unit groups, a freeze-dried preparation containing 5 or 100 units of teriparatide acetate per vial was dissolved in 1 mL of physiological saline solution, and the entire volume of solution was administered. Both the 5 and 100 unit groups were administered two tablets of a calcium agent (containing 500 mg of precipitated calcium carbonate [200 mg as calcium] per tablet) once a day.

The osteoporosis patients were classified as condition shown in Table 1 based on the bone fracture risk factors present, as in Non-patent Reference 13, and compared. High-risk patients were defined as having all three factors involving age, prevalent vertebral fractures, and bone density or bone atrophy; low-risk patients were defined as other than the above.

### [Table 1]

**Table 1. Classification by bone fracture risk factors**

| Risk factor | High-risk patients | Low-risk patients |
|---|---|---|
| Age | 65 years or older | Less than 65 years |
| Prevalent vertebral fractures | One or more | None |
| Evaluation of bone density or bone atrophy* | Less than 80% of the young adult mean** or atrophy grade I or higher | 80% of the young adult mean or higher or normal atrophy grade |

| | | |
|---|---|---|
| *Bone density evaluated by x-ray **Young adult mean: Average bone density of 20 to 44-year-olds | | |

Tables 2 and 3 show the patient background. No statistically significant differences could be found in the background of the two groups (p < 0.05).

### [Table 2]

**Table 2. Patient background of the 5 and 100 unit groups in high-risk patients**

| Mean±standard deviation | | | | |
|---|---|---|---|---|
| Group | No. of subjects | Age (years) | No. of prevalent vertebral fractures | Lumbar spine bone mineral density (%*) |
| 5 unit group | 64 | 73.9±4.6 | 2.3±1.2 | 63.4±11.6 |
| 100 unit group | 52 | 73.9±5.3 | 2.1±1.2 | 67.9±15.5 |

| | | | | |
|---|---|---|---|---|
| *Value taking the young adult mean as 100% | | | | |

### [Table 3]

**Table 3. Patient background of the 5 and 100 unit groups in low-risk patients**

| Mean±standard deviation | | | | |
|---|---|---|---|---|
| Group | No. of subjects | Age (years) | No. of prevalent vertebral fractures | Lumbar spine bone mineral density (%*) |
| 5 unit group | 10 | 57.9±5.1 | 1.8±0.9 | 68.3±8.8 |
| 100 unit group | 11 | 61.5±2.4 | 2.4±1.2 | 59.9±1.9 |

| | | | | |
|---|---|---|---|---|
| *Value taking the young adult mean as 100% | | | | |

The concomitant use of calcitonin preparations, active vitamin D₃ preparations, vitamin K preparations, ipriflavone preparations, bisphosphonate preparations, estrogen preparations, protein anabolic hormone preparations, calcium preparations prescribed by a physician (however, excluding the above calcium agent administered in a dose of two tablets once a day), and other drugs that might affect bone metabolism was prohibited during the administration period. The Lumbar spine bone mineral density and occurrence of bone fractures were checked as bone assessments. For the Lumbar spine bone mineral density, the bone density of the second to fourth lumbar spine was measured at the start and every six months thereafter using dual-energy x-ray absorptiometry (DXA). For the frequency of bone fractures, x-rays were taken from the front and side of the fourth thoracic to the fifth lumbar spine at the start and every six months thereafter, and new vertebral fractures were assessed by comparison of the roentgen films at the start and at each point in time thereafter using the method of Genant et al. (Non-patent Reference 14) as a reference. In locations other than the vertebrae, fractures were evaluated by checking by roentgen films. Blood samples were also taken from all patients at the beginning and during administration, and the general laboratory test values, including the calcium concentration, were measured. The administration period in high-risk patients (DXA and new vertebral fractures were assessed collectively at the center; fractures of other than vertebrae were assessed by the investigator based on the roentgen films) was 85.1±20.8 weeks in the 5 unit group and 83.7±19.8 weeks in the 100 unit group. No significant difference could be found between the two groups (p < 0.05). The administration period in low-risk patients was 72.7±19.4 weeks in the 5 unit group and 88.3±21.3 weeks in the 100 unit group. No significant difference could be found between the two groups (p < 0.05).

Tables 4 and 5 show the changes in the vertebral bone density by group in high-risk patients and low-risk patients. In the high-risk patients, a significant increase in the bone density of the 100 unit group was found in comparison to at the start of administration, and the value was also significantly higher than that of the 5 unit group (p < 0.05). On the other hand, no significant differences could be found in comparison with the start of administration and in comparison between groups in the low-risk patients (p > 0.05).

### [Table 4]

### [Table 4]

**Table 4. Lumbar spine bone mineral density situation (%change) in high-risk patients**

| Mean±standard deviation | | | | | |
|---|---|---|---|---|---|
| Group | After 26 W | After 52 W | After 78 W | After 104 W | Final check |
| 5 unit group | -0.9±4.4 | -0.6±4.1 | 0.4±4.1 | 0.4±4.0 | -0.1±4.8 |
| 100 unit group | 3.3±4.5*# | 4.3±4.5*# | 4.0±4.5*# | 4.9±4.2*# | 4.5±4.6*# |

| | | | | | |
|---|---|---|---|---|---|
| * Difference from the start of administration p < 0.05 # Difference from the 5 unit group p < 0.05 | | | | | |

### [Table 5]

**Table 5. Lumbar spine bone mineral density situation (%change) in low-risk patients**

| Mean±standard deviation | | | | |
|---|---|---|---|---|
| Group | After 26 W | After 52 W | After 78 W | Final check |
| 5 unit group | -0.6±6.4 | -0.7±6.2 | 1.9±7.5 | -0.6±5.7 |
| 100 unit group 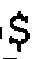 | 3.2 | 6.1 | 12.0 | 12.0 |

| | | | | |
|---|---|---|---|---|
| 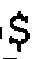 The bone density was measured in only one patient an the value is sown as a reference. | | | | |

Table 6 and 7 show the results on the occurrence of new vertebral fractures by group in high-risk and low-risk patients. In high-risk patients, the occurrence of fractures was significantly lower in the 100 unit group than in the 5 unit group (p < 0.05). On the other hand, no significant difference could be found between groups in the low-risk patients (p > 0.05).

### [Table 6]

**Table 6. Situation of new vertebral fractures in high-risk patients**

| Group | No. with fractures (persons) | No. of vertebral fractures | Difference between groups |
|---|---|---|---|
| 5 unit group | 13 | 22 | p < 0.05 |
| 100 unit group | 3 | 3 | |

### [Table 7]

**Table 7. Situation of new vertebral fractures in low-risk patients**

| Group | No. with fractures (persons) | No. of vertebral fractures | Difference between groups |
|---|---|---|---|
| 5 unit group | 1 | 1 | p > 0.05 |
| 100 unit group | 1 | 2 | |

Tables 8 and 9 show the results on the occurrence of new vertebral fractures by group every 26 weeks in high-risk patients and low-risk patients. In the high-risk patients, the occurrence of fractures was suppressed from after 26 weeks in the 100 unit group in comparison to the 5 unit group. On the other hand, no significant difference could be found between groups in the low-risk patients.

### [Table 8]

**Table 8. Situation of new vertebral fractures every 26 weeks in high-risk patients**

| | 5 unit group | | | | 100 unit group | | | |
|---|---|---|---|---|---|---|---|---|
| | No. evaluated | No. with fractures | Incidence (%) | No. of fractures | No. evaluated | No. with fractures | Incidence (%) | No. of fractures |
| After 26 W | 63 | 6 | 9.5 | 8 | 51 | 1 | 2.0 | 1 |
| After 52 W | 63 | 7 | 11.1 | 9 | 51 | 1 | 2.0 | 1 |
| After 78 W | 57 | 4 | 7.0 | 4 | 45 | 1 | 2.2 | 1 |
| After 104 W | 35 | 1 | 2.9 | 1 | 25 | 0 | -- | 0 |
| After 130 W | 10 | 0 | -- | 0 | 5 | 0 | -- | 0 |

### [Table 9]

**Table 9. Situation of new vertebral fractures every 26 weeks in low-risk patients**

| | 5 unit group | | | | 100 unit group | | | |
|---|---|---|---|---|---|---|---|---|
| | No. evaluated | No. with fractures | Incidence (%) | No. of fractures | No. evaluated | No. with fractures | Incidence (%) | No. of fractures |
| After 26 W | 21 | 0 | -- | 0 | 12 | 1 | 8.3 | 2 |
| After 52 W | 21 | 1 | 4.8 | 1 | 12 | 0 | -- | 0 |
| After 78 W | 16 | 0 | -- | 0 | 12 | 0 | -- | 0 |
| After 104 W | 2 | 0 | -- | 0 | 6 | 0 | -- | 0 |
| After 130 W | 2 | 0 | -- | 0 | 2 | 0 | -- | 0 |

Tables 10 and 11 show the results on the occurrence of fractures at sites other than the vertebrae by group in high-risk patients and low-risk patients. In the high-risk patients, the occurrence of fractures was significantly lower in the 100 unit group than in the 5 unit group. On the other hand, no significant difference could be found between groups in the low-risk patients.

### [Table 10]

**Table 10. Situation of fractures of sites other than the vertebrae in high-risk patients**

| Group | No. with fractures (persons) | Site | Difference between groups |
|---|---|---|---|
| 5 unit group | 6 | Right clavicle Proximal phalanx of the 5^{th} toe of the right foot Left 5^{th} rib Right 10^{th}, 11^{th} ribs Left pubis Right 6^{th} rib | p < 0.05 |
| 100 unit group | 1 | Neck of proximal phalanx of the 2^{nd} toe on the left | |

### [Table 11]

**Table 11. Situation of fractures of sites other than the vertebrae in low-risk patients**

| Group | No. with fractures (persons) | Site | Difference between groups |
|---|---|---|---|
| 5 unit group | 1 | 5^{th} toe of right foot | p > 0.05 |
| 100 unit group | 0 | | |

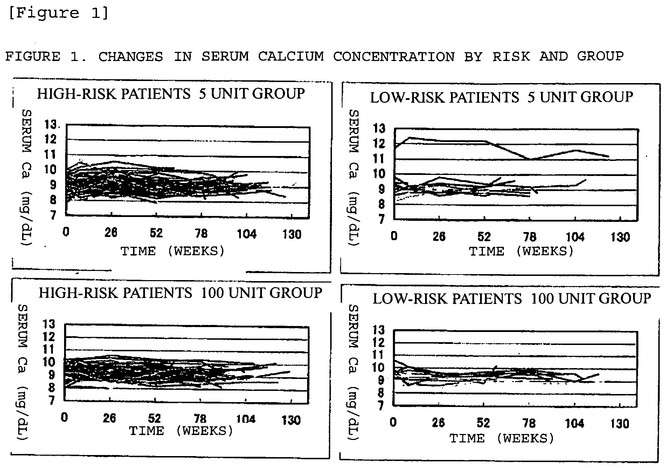

Figure 1 shows the results on the changes in the serum calcium concentration by group in high-risk patients and low-risk patients. No hypercalcemia was found in any case, except for one low-risk patient in the 5 unit group who had a high value from before the start of drug administration, in the results of the laboratory test values obtained using the sampled blood, and no tendency for the serum calcium to rise was seen.

As we understand from the above tables, subcutaneous administration of 100 units of teriparatide acetate once a week significantly increased the bone density of the lumbar spine and inhibited new vertebral fractures in primary osteoporosis patients having risk factors for new fractures. In other words, administration of 100 units of teriparatide acetate once a week was confirmed to provide a useful osteoporosis therapeutic agent and agent to inhibit or prevent bone fractures in the patients of the present invention at high risk for new fractures.

The administration of teriparatide acetate once a week of the present invention also did not cause hypercalcemia at either dose during the administration period and appeared to be more useful than the known daily administration of teriparatide acetate.

### (Working Example 2)

The test drug (one vial; injectable freeze-dried preparation containing 200 units of teriparatide acetate per vial), prepared by the method of Takai (Patent References 4 and 5 and Non-patent Reference 11), and control (one vial; placebo preparation substantially free of teriparatide acetate per vial) were each dissolved at the time of use in 1 mL of physiological saline solution and administered intermittently at a frequency of once a week over a span of 72 weeks to male and female high-risk patients diagnosed with primary osteoporosis.

The above patients also took two tablets of a calcium agent once a day after dinner. This calcium preparation was a soft chewable preparation that contained 610 mg of calcium, 400 IU of vitamin D₃, and 30 mg of magnesium in two tablets. The preparation contained precipitated calcium carbonate, magnesium carbonate, cholecalciferol (vitamin D₃), and the like as components and is marketed under the trade name "New Calcichew (trademark) D₃" (sold by: Daiichi Sankyo Health Care, manufactured and sold by: Nitto Pharmaceutical Industries, Ltd.).

Furthermore, the above patients were all outpatients who were able to walk on their own to whom (1) through (19) below did not pertain.
(1) Patients diagnosed with secondary osteoporosis due to a specific cause. Here, specific causes include endocrine (hyperthyroidism, gonadal failure, Cushing's syndrome), nutritional (scurvy, other (protein deficiency, vitamin A or D excess)), drugs (adrenocortical hormones, methotrexate (MTX), heparin, aromatase inhibitors, GnRH agonists), immobility (systemic (bed rest, paraplegia, airplane travel), localized (after bone fracture and the like)), congenital (osteogenesis imperfecta, Marfan's syndrome and the like), other (rheumatoid arthritis, diabetes, liver disease, gastrointestinal disease (gastric resection) and the like).
(2) Patients having specific diseases other than osteoporosis that present bone loss. Here, specific diseases include various osteomalacias, primary, secondary hyperparathyroidism, bone metastases of malignant tumors, multiple myeloma, vertebral hemangioma, Pott's disease, pyogenic spondylitis, and others.
(3) Patients having specific x-ray findings that might affect the strength of the vertebrae. Here, specific means the bridging of six or more consecutive vertebrae, severe ossification of the ligaments surrounding the vertebrae, severe spinal deformity in the spinal column, and having undergone surgery on the vertebrae.
(4) Patients who wear a corset that covers all of the thoracic and lumbar spine.
(5) Patients administered bisphosphonate preparations within 52 weeks (364 days) of giving consent.
(6) Patients being administered the following osteoporosis therapeutic agents at the time of consent (however, these can be selected as subjects if a washout period of 8 weeks (56 days) or longer is possible before beginning treatment): Calcitonin preparations, active vitamin D₃ preparations, vitamin K preparations, ipriflavone preparations, estrogen preparations, SERM preparations, protein anabolic hormone preparations.
(7) Patients with bronchial asthma or a tendency to rash (erythema, urticaria, and the like), and other such hypersensitivity symptoms.
(8) Patients with a history of hypersensitivity to PTH preparations.
(9) Patients with Paget's disease of the bone.
(10) Patients with existing malignant bone tumors or having had malignant tumors within the past five years.
(11) Patients with multiple myeloma.
(12) Patients with a history of internal or external radiation therapy of the skeleton.
(13) Patients with a serum calcium value of 11.0 mg/dL or higher.
(14) Patients with an alkaline phosphatase value two or more times the upper limit of the standard value.
(15) Patients with serious kidney disease, liver disease, or heart disease. The criteria for each disease are as follows:
   Kidney disease: Serum creatinine value of 2 mg/dL or higher
   Liver disease: AST (GOT) or ALT (GPT) value of 2.5 times the upper limit of the standard value or higher or 100 IU/L or higher
   Heart disease: Assessed using grade 2 shown in the "Severity classification criteria for side effects of pharmaceutical products (June 29, 1992 Drug Safety Notification No. 80)" as a reference.
(16) Patients judged to have low reliability in medical interview (definitely exclude at least patients with dementia).
(17) Patients who have been administered other test drugs within 26 weeks (182 days) before consent.
(18) Patients who have been administered PTH preparations in past trials.
(19) Other patients deemed unsuitable for the trial by the investigator.

Administration of any of the following drugs of (1) through (6) to the above patients was prohibited from the time of consent to the trial until the end of the trial.
(1) Osteoporosis therapeutic agents other than teriparatide acetate (specifically, bisphosphonate preparations, calcitonin preparations, active vitamin D₃ preparations, calcium agents (however, excluding the above calcium agent taken once a day after dinner), vitamin K preparations, ipriflavone preparations, estrogen preparations, SERM preparations, and anabolic hormone preparations)
(2) Adrenocortical hormone preparations (however, when the average weekly dose exceeds 5 mg/day, the daily dose exceeds 10 mg/day, or the total dose exceeds 450 mg, calculated for prednisolone, administered intramuscularly, intravenously, or orally)
(3) Aromatase inhibitors
(4) GnRH agonists
(5) Other test drugs

The numbers of patients administered the test drug and control were 290 (also sometimes referred to as the test drug group in the working example) and 288 (also sometimes referred to as the control group in the working example), respectively. The total number administered was 578. However, the number in each group sometimes differs depending on the type of test and is expressed, for example, by (n = **) or the number evaluated, or the like.

Bone density, bone geometry, and incidence of bone fractures were checked as bone assessments.

For the bone density of the lumbar spine, the density of the second to fourth lumbar spine was measured at the start and every 24 weeks thereafter using dual-energy x-ray absorptiometry (DXA).

For the bone density of the femur, only the left side of the proximal part of the femur, rotated 20°, was measured at the start and every 24 weeks thereafter using dual-energy x-ray absorptiometry (DXA).

The DXA geometry was evaluated by the femur bone density data measured by the investigator at the start and every 24 weeks thereafter.

For the CT geometry, the proximal part of the femur was measured at the start and after 48 and 72 weeks using multislice CT scan.

The incidence of bone fractures was checked by frontal and lateral x-rays of the fourth thoracic to the fourth lumbar spine taken at the start and every 24 weeks thereafter. The roentgen films at the start and at each point in time thereafter were compared, using the method of Genant et al. (Non-patent Reference 14) as a reference, and new and worsening vertebral fractures were evaluated. Locations other than the vertebrae were evaluated by checking by roentgen film (DXA, bone geometry, and new and worsening vertebral fractures were assessed collectively at the center; fractures of other than vertebrae were assessed by the investigator based on the roentgen films).

### (A) Efficacy of the test drug against multiple vertebral fractures

Here, "multiple vertebral fractures" was defined as two or more new vertebral fractures. When the incidence of multiple vertebral fractures (number of cases) was compared in the test drug group (n = 261) and control group (n = 281) after 72 weeks of administration, it was 2.1% (6 cases) in the control group and 0.8% (2 cases) in the test drug group. In other word, the test drug demonstrated an inhibitory or preventative effect against multiple vertebral fractures.

The number of cases with each number of fractures appears in the table below.

**[Table 12]**

| No. of fractures | New vertebral fractures | | | |
|---|---|---|---|---|
| | Test drug group (n = 261) | | Control group (n = 281) | |
| | No. of subjects | Incidence (%) | No. of subjects | Incidence (%) |
| None | 254 | 97.3 | 244 | 86.8 |
| One | 5 | 1.9 | 31 | 11 |
| Two | 2 | 0.8 | 4 | 1.4 |
| Three | 0 | 0.0 | 1 | 0.4 |
| Four | 0 | 0.0 | 1 | 0.4 |
| Five or more | 0 | 0.0 | 0 | 0.0 |

### (B) Efficacy of the test drug in primary osteoporosis patients taking glucocorticoid

The effect of administration of the test drug to primary osteoporosis patients taking glucocorticoid was studied. The results were as in the table below. The test drug was shown to be effective in primary osteoporosis patients taking glucocorticoid.

**[Table 13]**

| | Change in bone density (%) | | | | | | | | | | | |
|---|---|---|---|---|---|---|---|---|---|---|---|---|
| glucocorticoid | Lumbar spine | | | | Neck of femur | | | | Proximal femur | | | |
| Time after administration | 0 W | 24 W | 48 W | 72 W | 0 W | 24 W | 48 W | 72 W | 0 W | 24 W | 48 W | 72 W |
| Test drug group (n = 14, 18, 18) | 0.00 | 3.50 | 5.97 | 6.82 | 0.00 | 2.53 | 2.79 | 2.44 | 0.00 | 2.46 | 3.39 | 3.06 |
| Control group (n = 17, 21, 21) | 0.00 | 1.26 | 1.91 | 1.23 | 0.00 | -2.51 | -1.52 | -1.74 | 0.00 | -0.86 | -0.8 | 0.81 |

**[Table 14]**

| glucocorticoid | Existence of new vertebral fractures | | | Total |
|---|---|---|---|---|
| | Discontinued | Fracture: yes | Fracture: no | |
| Test drug group | 4 | 0 | 29 | 33 |
| Control group | 3 | 3 | 24 | 30 |

Since glucocorticoid are drugs that cause secondary osteoporosis, the above results suggest that the test drug may be effective against secondary osteoporosis caused by drugs that trigger secondary osteoporosis such as glucocorticoid.

### (C) Efficacy of the test drug on three parts of the femur

The effects of the test drug on three parts of the femur (neck of the femur, trochanter, and shaft of the femur) were studied using ordinary CT scans. The results were as in the table below. The test drug was shown to be effective on each part of the femur.

**[Table 15]**

| (C-1)Effect on the neck of the femur | | | | | | |
|---|---|---|---|---|---|---|
| Neck of femur | (At start →48 W →72 W) | Baseline | Change from baseline after 48 weeks (%) | p value (between groups (* P < 0.05) | Change from baseline after 72 weeks (%) | p value (between groups (* P < 0.05) |
| Volume bone mineral density (total) [vBMD total] | Test drug group (n = 30→28→22) | 221.38 | 0.92 | 0.1155 | 0.15 | 0.1658 |
| | Control group (n = 37→33→30) | 227.98 | -0.72 | | -1.23 | |
| Volume bone mineral density (cortical bone) [vBMD cortical] | Test drug group (n = 30→28→22) | 665.02 | -0.77 | 0.5638 | -1.14 | 0.6852 |
| | Control group (n = 37→33→30) | 676.84 | -0.22 | | -0.82 | |
| Buckling ratio | Test drug group (n = 30→28→22) | 14.01 | -3.48 | *0.0084 | -3.40 | *0.012 |
| | Control group (n = 37→33→30) | 13.44 | 1.27 | | 1.85 | |
| Maximum section modulus (SM (Z min)) | Test drug group (n = 30→28→22) | 0.3851 | 3.43 | 0.0819 | 1.87 | 0.5837 |
| | Control group (n = 37→33→30) | 0.3793 | -0.27 | | 0.62 | |

**[Table 16]**

| (C-2) Effect on the trochanter | | | | | | |
|---|---|---|---|---|---|---|
| Trochanter | (At start →48 W →72 W) | Baseline | Change from baseline after 48 weeks (%) | p value (between groups (* P < 0.05) | Change from baseline after 72 weeks | p value (between groups (* P < 0.05) |
| Volume bone mineral density (total) [vBMD total] | Test drug group (n = 30→28→22) | 186.45 | 1.36 | *0.0086 | 0.95 | 0.0802 |
| | Control group (n = 36→32→30) | 196.10 | -1.50 | | -1.47 | |
| Volume bone mineral density (cortical bone) [vBMD cortical] | Test drug group (n = 30→28→22) | 638.98 | -0.49 | 0.8300 | -1.49 | 0.1653 |
| | Control group (n = 36→32→30) | 646.03 | -0.34 | | -0.61 | |
| Buckling ratio | Test drug group (n = 30→28→22) | 19.64 | 1.59 | 0.3015 | 1.27 | 0.7681 |
| | Control group (n = 36→32→30) | 19.26 | 4.26 | | 2.13 | |
| Maximum section modulus (SM (Z min )) | Test drug group (n = 30→28→22) | 0.6989 | 5.19 | 0.4324 | 3.20 | 0.6567 |
| | Control group (n = 36→32→30) | 0.7295 | 2.38 | | 1.81 | |

**[Table 17]**

| (C-3) Effect on the shaft of the femur | | | | | | |
|---|---|---|---|---|---|---|
| Shaft of femur | (At start →48 W →72 W) | Baseline | Change from baseline after 48 weeks (%) | p value (between groups (* P < 0.05) | Change from baseline after 72 weeks (%) | p value (between groups (* P < 0.05) |
| Volume bone mineral density (total) [vBMD total] | Test drug group (n = 29→28→21) | 463.59 | 1.03 | 0.3521 | 1.25 | 0.0613 |
| | Control group (n = 37→33→30) | 482.05 | -0.22 | | -1.43 | |
| Volume bone mineral density (cortical bone) [vBMD cortical] | Test drug group (n = 29→28→21) | 880.91 | 0.59 | 0.7796 | 0.18 | 0.2703 |
| | Control group (n = 37→33→30) | 892.97 | 0.33 | | -0.87 | |
| Buckling ratio | Test drug group (n = 29→28→21) | 3.64 | -0.66 | 0.3977 | -3.54 | *0.0008 |
| | Control group (n = 37→33→30) | 3.39 | 0.92 | | 1.91 | |
| Maximum section modulus (SM (Z min )) | Test drug group (n = 29→28→21) | 0.9015 | 1.28 | 0.1499 | 2.69 | 0.1604 |
| | Control group (n = 37→33→30) | 0.9343 | -0.78 | | 0.32 | |

### (D) Study of prescriptions for nausea and vomiting associated with administration of the test drug

The administration period and efficacy of various drugs to treat nausea and vomiting associated with administration of the test drug were studied.

**[Table 18]**

| Event | Treated (total no. of subjects) | Name of drug used in treatment | Before or after test drug | Effective or ineffective |
|---|---|---|---|---|
| | | Gaster D | Unknown | Effective |
| | | Gaster D, Nauzelin | Unknown | Effective |
| | | Nauzelin | Before | Unknown (some days effective, some days not) |
| | | Novamine | Before | Ineffective |
| | | Primperan tablet | Before | Ineffective |
| | | Primperan | Before | Ineffective |
| | | Primperan | Before | Some effect |
| | | Nauzelin (taken after breakfast, lunch, and dinner) | Before | Effective |
| Nausea | 20 | Takepron OD tablet | After | Ineffective |
| | | Gaster D tablet | After | Ineffective |
| | | Gasmotin tablet | After | Effective |
| | | Nauzelin | After | Effective |
| | | Primperan | After | Some effect |
| | | Primperan | After | Effective |
| | | Primperan tablet | After | Effective |
| | | Takepron OD | After | Effective |
| | | Primperan tablet, primperan injection | After | Effective |
| | | Primperan, Takepron OD | After | Effective |
| | | Gaster D20, Nauzelin | After | Effective |
| | | Rokushingan | After | Effective |
| Vomiting | | Nauzelin (taken after breakfast, lunch, and dinner) | Before | Effective |

As shown above, primperan, Nauzelin, Gaster D, Gasmotin, Takepron OD, and rokushingan were effective. Nauzelin, Gasmotin, and rokushingan were especially preferred.

### (E) Evaluation of the effects of the types and existence of complications on the effects of the test drug

Some of the above patients had complications. The effects of the type (diabetes, hypertension, hyperlipidemia) and existence of complications on the effects of the test drug were therefore evaluated. The results were as in the table below. The test drug was clarified to inhibit the development of new vertebral fractures from after 24 weeks of administration regardless of the type or existence of complications.

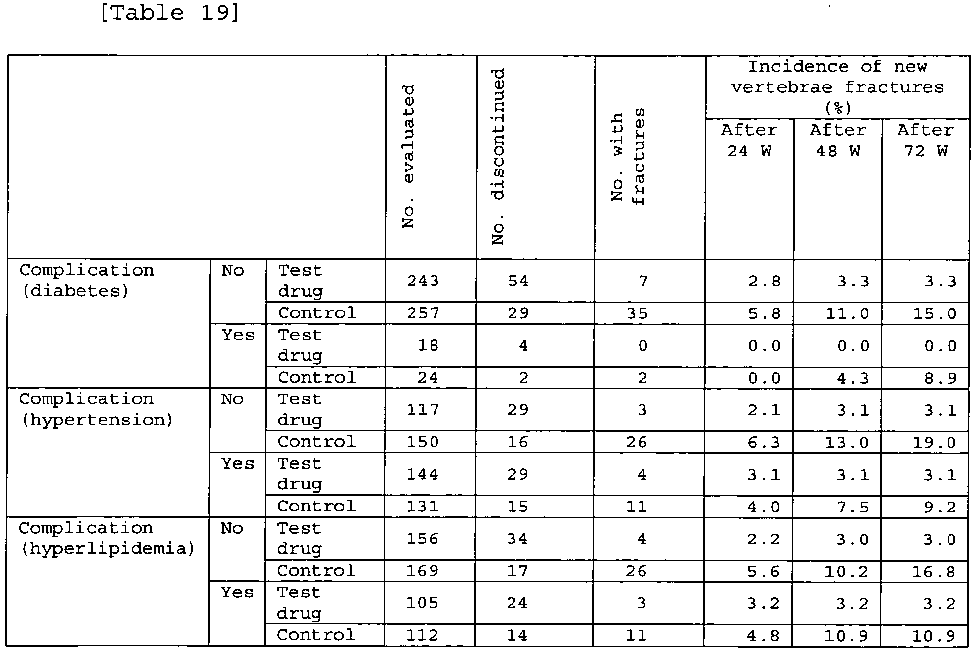

Diabetic osteoporosis in which diabetes as the underlying disease is one form of secondary osteoporosis, but the fact that the test drug was found to have an effect in primary osteoporosis patients having diabetes as a complication suggests the possibility that the test drug will also have a therapeutic effect in diabetic osteoporosis.

### (F) Efficacy of the test drug on worsening bone fractures

The efficacy of the test drug on worsening bone fractures was studied. The results were as in the table below. The test drug was shown to be effective on worsening bone fractures.

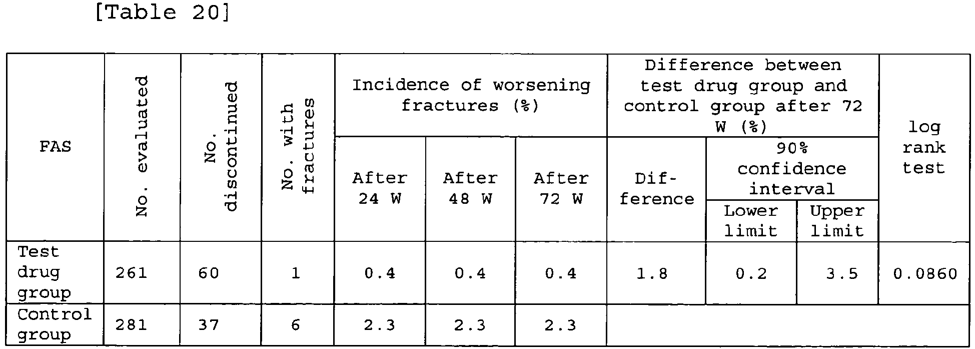

### (G) Evaluation of the effect of a history of taking other osteoporosis therapeutic agents on the efficacy of the test drug

As was stated above, the above patients were prohibited as a general rule from being administered any osteoporosis therapeutic agents other than teriparatide acetate from the time of consenting to the trial until the end of the trial. However, there were also patients who took other osteoporosis therapeutic agents under specific conditions before consenting to the trial. The effects of a history of taking other osteoporosis therapeutic agents on the efficacy of the test drug were therefore evaluated from the viewpoints of the incidence of new vertebral fractures and the percentage change in bone density.

The table below shows the results obtained by evaluating the incidence of new vertebral fractures. In the table, the incidence of fractures was 2.9% in the test drug group and 16.1% in the control group after 72 weeks of administration in patients with a history of taking other osteoporosis therapeutic agents. The incidence of bone fractures was 3.2% in the test drug group and 12.9% in the control group in patients with no such history. In other words, the test drug was clarified to have higher efficacy in patients with a history of taking other osteoporosis therapeutic agents than in those with no such history.

**[Table 21]**

| | | No. evaluated | No. discontinued | No. with fractures | Incidence of new vertebral fractures (%) | | |
|---|---|---|---|---|---|---|---|
| | | | | | After 24 W | After 48 W | After 72 W |
| Prior osteoporosis treatment (no) | Test drug group | 139 | 31 | 4 | 2.4 | 3.2 | 3.2 |
| | Control group | 142 | 13 | 17 | 6.7 | 9.8 | 12.9 |
| Prior osteoporosis treatment (yes) | Test drug group | 122 | 27 | 3 | 2.9 | 2.9 | 2.9 |
| | Control group | 139 | 18 | 20 | 3.8 | 11.2 | 16.1 |

The table below shows the results obtained by evaluating the percentage change in bone density. In the table, the increase in Lumbar spine bone mineral density after 48 weeks of administration of the test drug was remarkable even in patients with a history of taking other osteoporosis therapeutic agents. In particular, a remarkable increase in Lumbar spine bone mineral density was seen as early as after 24 weeks of administration in the test drug group when the other osteoporosis therapeutic agents were calcium L-aspartate, elcatonin, Alfacalcidol, menatetrenone, and calcitriol. It is particularly noteworthy that remarkable increases in the bone density of the neck of the femur and proximal femur were seen after 72 weeks of administration of the test drug when the other osteoporosis therapeutic agents were calcium L-aspartate and elcatonin. When the other osteoporosis therapeutic agent was elcatonin in particular, it deserves special mention that a vast increase was seen in the bone density of the proximal femur as early as after 24 weeks of administration of the test drug.

**[Table 22]**

| | Percentage change in bone density (%) | | | | | | | | | | | |
|---|---|---|---|---|---|---|---|---|---|---|---|---|
| Calcium L-aspartate | Lumbar spine | | | | Neck of femur | | | | Proximal femur | | | |
| Week | 0 W | 24 W | 48 W | 72 W | 0 W | 24 W | 48 W | 72 W | 0 W | 24 W | 48 W | 72 W |
| PTH200 group (n = 17, 23, 23) | 0.00 | 3.78 | 6.16 | 5.85 | 0.00 | 1.44 | 1.88 | 4.17 | 0.00 | 1.76 | 2.28 | 3.69 |
| P group (n = 20, 24, 24) | 0.00 | -0.31 | -1.01 | -1.52 | 0.00 | 0.57 | 0.83 | 1.61 | 0.00 | 0.71 | -0.18 | -0.17 |

| Alfacalcidol | Lumbar spine | | | | Neck of femur | | | | Proximal femur | | | |
|---|---|---|---|---|---|---|---|---|---|---|---|---|
| Week | 0 W | 24 W | 48 W | 72 W | 0 W | 24 W | 48 W | 72 W | 0 W | 24 W | 48 W | 72 W |
| PTH200 group (n = 41, 51, 51) | 0.00 | 3.11 | 5.47 | 6.47 | 0.00 | 1.64 | 0.85 | 2.19 | 0.00 | 2.77 | 2.31 | 2.31 |
| P group (n = 37, 46, 45) | 0.00 | -0.65 | -1.01 | -0.69 | 0.00 | -0.28 | -0.16 | -0.96 | 0.00 | 0.11 | -0.89 | -1.05 |

| Raloxifene hydrochloride | Lumbar spine | | | | Neck of femur | | | | Proximal femur | | | |
|---|---|---|---|---|---|---|---|---|---|---|---|---|
| Week | 0 W | 24 W | 48 W | 72 W | 0 W | 24 W | 48 W | 72 W | 0 W | 24 W | 48 W | 72 W |
| PTH200 group (n = 18, 21, 21 | 0.00 | 1.77 | 4.96 | 4.59 | 0.00 | 0.21 | 1.03 | -0.51 | 0.00 | 2.44 | 2.26 | 3.03 |
| P group (n = 22, 24, 24) | 0.00 | -0.33 | -1.74 | -1.89 | 0.00 | -0.61 | -0.61 | 0.58 | 0.00 | 0.04 | 0.45 | 0.62 |

| Elcatonin | Lumbar spine | | | | Neck of femur | | | | Proximal femur | | | |
|---|---|---|---|---|---|---|---|---|---|---|---|---|
| Week | 0 W | 24 W | 48 W | 72 W | 0 W | 24 W | 48 W | 72 W | 0 W | 24 W | 48 W | 72 W |
| PTH200 group (n = 22, 28, 28) | 0.00 | 3.95 | 3.59 | 5.36 | 0.00 | 1.62 | 0.72 | 3.15 | 0.00 | 3.86 | 3.44 | 4.41 |
| P group (n = 20, 26, 26) | 0.00 | -0.29 | 0.20 | 0.95 | 0.00 | -1.35 | 0.95 | 0.29 | 0.00 | -0.73 | -0.95 | 0.10 |

| | Percentage change in bone density (%) | | | | | | | | | | | |
|---|---|---|---|---|---|---|---|---|---|---|---|---|
| Menatetrenone | Lumbar spine | | | | Neck of femur | | | | Proximal femur | | | |
| Week | 0 W | 24 W | 48 W | 72 W | 0 W | 24 W | 48 W | 72 W | 0 W | 24 W | 48 W | 72 W |
| PTH200 group (n = 5,5,5) | 0.00 | 2.70 | 3.04 | 4.32 | 0.00 | 0.72 | -3.18 | -0.78 | 0.00 | 0.60 | -0.56 | -1.40 |
| P group (n = 4,6,6) | 0.00 | -1.03 | 2.47 | -1.53 | 0.00 | -1.16 | 1.03 | -4.70 | 0.00 | -0.34 | -2.43 | -3.78 |

| Calcium lactate | Lumbar spine | | | | Neck of femur | | | | Proximal femur | | | |
|---|---|---|---|---|---|---|---|---|---|---|---|---|
| Week | 0 W | 24 W | 48 W | 72 W | 0 W | 24 W | 48 W | 72 W | 0 W | 24 W | 48 W | 72 W |
| PTH200 group (n = 7, 5, 5) | 0.00 | 1.96 | 3.45 | 4.93 | 0.00 | 0.30 | -4.35 | -2.53 | 0.00 | 1.78 | -1.68 | -3.20 |
| P group (n = 6, 9, 9) | 0.00 | -0.88 | -0.17 | 0.43 | 0.00 | -2.19 | 0.83 | -3.00 | 0.00 | 0.39 | 1.15 | -1.58 |

| Calcitriol | Lumbar spine | | | | Neck of femur | | | | Proximal femur | | | |
|---|---|---|---|---|---|---|---|---|---|---|---|---|
| Week | 0 W | 24 W | 48 W | 72 W | 0 W | 24 W | 48 W | 72 W | 0 W | 24 W | 48 W | 72 W |
| PTH200 group (n = 5, 6, 6) | 0.00 | 3.50 | 4.40 | 6.38 | 0.00 | -0.60 | -4.03 | -3.53 | 0.00 | -1.40 | - 1.05 | -0.04 |
| P group (n = 11, 13, 13) | 0.00 | 0.70 | 0.49 | - 0.72 | 0.00 | - 1.26 | -1.25 | -3.81 | 0.00 | 0.55 | -0.01 | - 0.43 |

The effects of a history of taking other osteoporosis therapeutic agents on the efficacy of the test drug were also evaluated in detail from the standpoint of the incidence of new vertebral fractures for each of the other osteoporosis therapeutic agents. The results are shown in the table below. As is understood from the table, remarkable inhibition of new fractures due to administration of the test drug was seen in patients with a history of taking other osteoporosis therapeutic agents other than calcitriol.

**[Table 23]**

| Calcium L-aspartate | New vertebral fractures: yes or no | | | Total |
|---|---|---|---|---|
| | Discontinued | Yes | No | |
| Test drug group | 9 | 1 | 28 | 38 |
| Control group | 2 | 5 | 27 | 34 |

| Alfacalcidol | New vertebral fractures: yes or no | | | Total |
|---|---|---|---|---|
| | Discontinued | Yes | No | |
| Test drug group | 16 | 1 | 62 | 79 |
| Control group | 12 | 11 | 50 | 73 |

| Raloxifene hydrochloride | New vertebral fractures: yes or no | | | Total |
|---|---|---|---|---|
| | Discontinued | Yes | No | |
| Test drug group | 9 | 0 | 20 | 29 |
| Control group | 8 | 5 | 24 | 37 |

| Elcatonin | New vertebral fractures: yes or no | | | Total |
|---|---|---|---|---|
| | Discontinued | Yes | No | |
| Test drug group | 10 | 2 | 35 | 47 |
| Control group | 9 | 5 | 34 | 48 |

| Menatetrenone | New vertebral fractures: yes or no | | | Total |
|---|---|---|---|---|
| | Discontinued | Yes | No | |
| Test drug group | 1 | 0 | 6 | 7 |
| Control group | 3 | 3 | 4 | 10 |

| Calcium lactate | New vertebral fractures: yes or no | | | Total |
|---|---|---|---|---|
| | Discontinued | Yes | No | |
| Test drug group | 1 | 0 | 8 | 9 |
| Control group | 1 | 2 | 7 | 10 |

| Calcitriol | New vertebral fractures: yes or no | | | Total |
|---|---|---|---|---|
| | Discontinued | Yes | No | |
| Test drug group | 2 | 1 | 7 | 10 |
| Control group | 1 | 2 | 13 | 16 |

### (H) Efficacy and safety of the test drug for osteoporosis patients having renal dysfunction

The efficacy and safety of the test drug were studied in a group of osteoporosis patients having normal renal function, a group of osteoporosis patients having mild renal dysfunction, and a group of osteoporosis patients having moderate renal dysfunction.

### (H-1) Distribution of background factors of each group (details)

The group of osteoporosis patients having normal renal function was listed as "normal (≥80)," the group of osteoporosis patients having mild renal dysfunction as "mild impairment (≥50 to <80)," and the group of osteoporosis patients having moderate renal dysfunction as "moderate impairment (<50)." The test drug group was listed as the "PTH200 group" and the control group as the "P group." The group of osteoporosis patients having mild renal dysfunction and group of osteoporosis patients having moderate renal dysfunction are also sometimes listed together as "abnormal (<80)." Each patient was classified into the above groups based on their creatinine clearance. Specifically, creatinine clearance of 80 mL/min or higher was regarded as normal renal function, from 50 to less than 80 mL/min as mild renal dysfunction, and from 30 to less than 50 mL/min as moderate renal dysfunction.

### (H-1) Distribution of background factors of each group

The distribution of background factors of each group was as follows.

**[Table 24]**

| Ccr at start | Safety analysis set | Group | No. of cases | Average value | Standard deviation | Minimum value | Median value | Maximum value |
|---|---|---|---|---|---|---|---|---|
| Normal (≥80) | Age | PTH200 | 49 | 70.2 | 3.8 | 65 | 70.0 | 78 |
| | | P | 49 | 70.4 | 3.9 | 65 | 70.0 | 82 |
| | Height (cm) | PTH200 | 49 | 150.56 | 5.65 | 138.5 | 151.50 | 162.5 |
| | | P | 49 | 151.11 | 4.46 | 140.0 | 151.20 | 162.5 |
| | Weight (kg) | PTH200 | 49 | 57.81 | 5.42 | 47.5 | 57.30 | 70.5 |
| | | P | 49 | 57.51 | 7.73 | 41.5 | 56.50 | 80.1 |
| | BMI (kg/m²) | PTH200 | 49 | 25.57 | 2.77 | 20.5 | 25.70 | 35.5 |
| | | P | 49 | 25.22 | 3.57 | 17.7 | 24.80 | 37.2 |
| | Time after menopause (years) | PTH200 | 49 | 19.7 | 4.8 | 11 | 19.0 | 30 |
| | | P | 49 | 20.6 | 6.0 | 10 | 20.0 | 42 |
| | Prevalent vertebral fractures at enrollment (no.) | PTH200 | 49 | 2.0 | 1.2 | 1 | 1.0 | 5 |
| | | P | 49 | 1.7 | 0.8 | 1 | 1.0 | 4 |
| | Prevalent vertebral fractures before administration (no.) | PTH200 | 49 | 1.8 | 1.3 | 0 | 1.0 | 5 |
| | | P | 49 | 1.3 | 0.9 | 0 | 1.0 | 4 |
| | Lumbar spine bone mineral density before administration (YAM converted) (%) | PTH200 | 33 | 68.9 | 8.4 | 51 | 69.1 | 82 |
| | | P | 38 | 70.6 | 9.0 | 50 | 70.4 | 91 |
| | Neck of femur bone mineral density before administration (YAM converted) (%) | PTH200 | 37 | 73.3 | 8.7 | 57 | 74.4 | 94 |
| | | P | 37 | 72.6 | 9.3 | 48 | 73.3 | 92 |
| | Proximal femur total bone mineral density before administration (YAM converted) (%) | PTH200 | 37 | 75.4 | 11.4 | 51 | 76.0 | 96 |
| | | P | 36 | 79.5 | 9.0 | 57 | 80.6 | 97 |
| Mild impairment (≥50-<80) | Age | PTH200 | 160 | 74.3 | 4.7 | 65 | 74.0 | 85 |
| | | P | 151 | 74.5 | 4.6 | 65 | 74.0 | 90 |
| | Height (cm) | PTH200 | 160 | 147.57 | 5.14 | 136.2 | 147.35 | 166.0 |
| | | P | 151 | 147.41 | 5.15 | 134.5 | 147.0 | 160.4 |
| | Weight (kg) | PTH200 | 160 | 50.57 | 6.19 | 37.2 | 50.40 | 69.0 |
| | | P | 151 | 49.73 | 6.56 | 34.2 | 49.80 | 66.0 |
| | BMI (kg/m²) | PTH200 | 160 | 23.26 | 2.85 | 15.9 | 23.20 | 31.3 |
| | | P | 151 | 22.89 | 2.82 | 15.3 | 22.80 | 30.1 |
| | Time after menopause (years) | PTH200 | 160 | 24.9 | 5.6 | 11 | 24.0 | 39 |
| | | P | 151 | 25.2 | 5.6 | 12 | 25.0 | 43 |
| | Prevalent vertebral fractures at enrollment (no.) | PTH200 | 160 | 1.9 | 1.1 | 1 | 1.5 | 5 |
| | | P | 151 | 2.0 | 1.2 | 1 | 2.0 | 5 |
| | Prevalent vertebral fractures before administration (no.) | PTH200 | 160 | 1.8 | 1.4 | 0 | 1.0 | 8 |
| | | P | 151 | 1.7 | 1.3 | 0 | 1.0 | 6 |
| | Lumbar spine bone mineral density before administration (YAM converted) (%) | PTH200 | 91 | 68.4 | 9.4 | 51 | 67.7 | 99 |
| | | P | 84 | 67.2 | 11.4 | 42 | 67.9 | 98 |
| | Neck of femur bone mineral density before administration (YAM converted) (%) | PTH200 | 104 | 67.3 | 8.7 | 46 | 67.0 | 96 |
| | | P | 95 | 65.6 | 10.0 | 38 | 66.1 | 91 |
| | Proximal femur total bone mineral density before administration (YAM converted) (%) | PTH200 | 103 | 72.2 | 11.3 | 44 | 70.5 | 102 |
| | | P | 95 | 69.7 | 11.2 | 42 | 70.2 | 91 |
| Moderate impairment (<50) | Age | PTH200 | 68 | 80.5 | 5.5 | 65 | 80.0 | 93 |
| | | P | 78 | 80.3 | 5.5 | 65 | 81.0 | 95 |
| | Height (cm) | PTH200 | 68 | 144.79 | 6.57 | 124.3 | 145.00 | 158.0 |
| | | P | 78 | 143.99 | 6.14 | 133.0 | 143.50 | 159.0 |
| | Weight (kg) | PTH200 | 68 | 44.48 | 6.65 | 30.4 | 43.65 | 60.5 |
| | | P | 78 | 44.59 | 6.74 | 31.0 | 44.25 | 67.0 |
| | BMI (kg/m²) | PTH200 | 68 | 21.28 | 3.28 | 14.5 | 21.30 | 29.5 |
| | | P | 78 | 21.54 | 3.21 | 16.2 | 21.40 | 32.7 |
| | Time after menopause (years) | PTH200 | 68 | 31.0 | 6.7 | 12 | 30.0 | 46 |
| | | P | 78 | 30.5 | 6.7 | 12 | 31.0 | 48 |
| | Prevalent vertebral fractures at enrollment (no.) | PTH200 | 68 | 2.3 | 1.3 | 1 | 2.0 | 5 |
| | | P | 78 | 2.1 | 1.3 | 1 | 2.0 | 5 |
| | Prevalent vertebral fractures before administration (no.) | PTH200 | 68 | 2.3 | 1.5 | 0 | 2.0 | 6 |
| | | P | 78 | 1.8 | 1.3 | 0 | 1.5 | 5 |
| | Lumbar spine bone mineral density before administration (YAM converted) (%) | PTH200 | 30 | 65.9 | 13.5 | 31 | 64.3 | 102 |
| | | P | 39 | 71.1 | 11.6 | 51 | 72.8 | 102 |
| | Neck of femur bone mineral density before administration (YAM converted) (%) | PTH200 | 40 | 62.8 | 12.1 | 39 | 62.0 | 91 |
| | | P | 46 | 63.5 | 10.4 | 42 | 63.1 | 88 |
| | Proximal femur total bone mineral density before administration (YAM converted) (%) | PTH200 | 40 | 65.6 | 13.2 | 33 | 62.9 | 90 |
| | | P | 46 | 67.9 | 12.7 | 43 | 66.8 | 93 |

### (H-2) Efficacy of the test drug in each group (inhibition of bone fractures)

The test drug was clarified to have an inhibitory effect on new vertebral fractures in the group of osteoporosis patients having normal renal function and the groups of osteoporosis patients having renal dysfunction (mild and moderate).

**[Table 25]**

| | | | No. evaluated | No. discontinued | No. with fractures | Incidence of new vertebral fractures (%) | | |
|---|---|---|---|---|---|---|---|---|
| | | | | | | After 24 W | After 48 W | After 72 W |
| Creatinine clearance value at start | Normal (≥80) | PTH200 | 46 | 9 | 0 | 0.0 | 0.0 | 0.0 |
| | | P | 48 | 5 | 4 | 2.2 | 4.4 | 9.1 |
| | Abnormal (<80) | PTH200 | 202 | 47 | 6 | 2.8 | 3.4 | 3.4 |
| | | P | 223 | 25 | 31 | 6.2 | 12.2 | 15.3 |

### (H-3) Efficacy of the test drug in each group (increased bone density)

The test drug was clarified to have the effect of increasing the lumbar vertebral density in the group of osteoporosis patients having normal renal function, group of osteoporosis patients having mild renal dysfunction, and group of osteoporosis patients having moderate renal dysfunction.

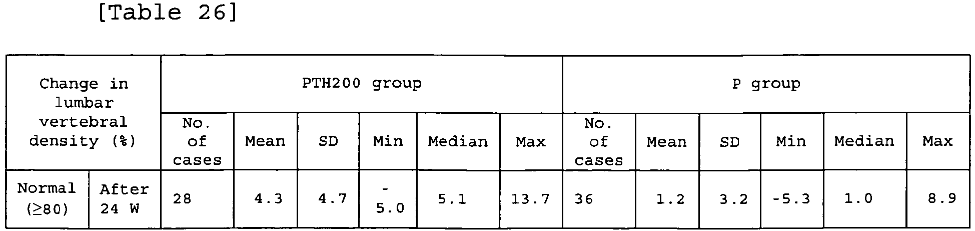

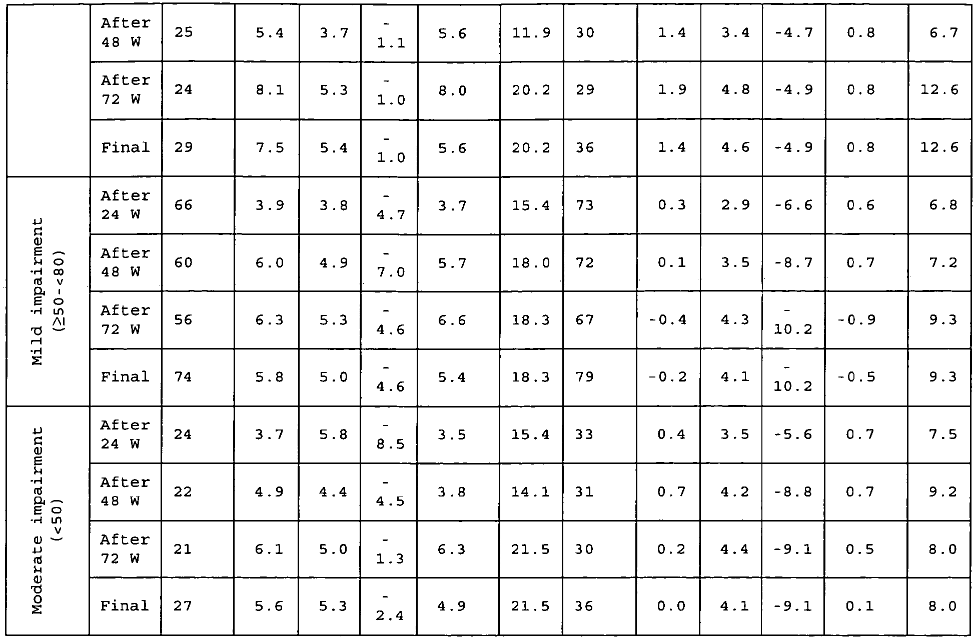

### (H-4) Safety of the test drug in each group (corrected serum calcium)

No significant differences could be found between any group administered the test drug and the control group as a result of administering the test drug to a group of osteoporosis patients having normal renal function, a group of osteoporosis patients having mild renal dysfunction, and a group of osteoporosis patients having moderate renal dysfunction. More specifically, the test drug was clarified to have equivalent safety in terms of the serum calcium in all groups.

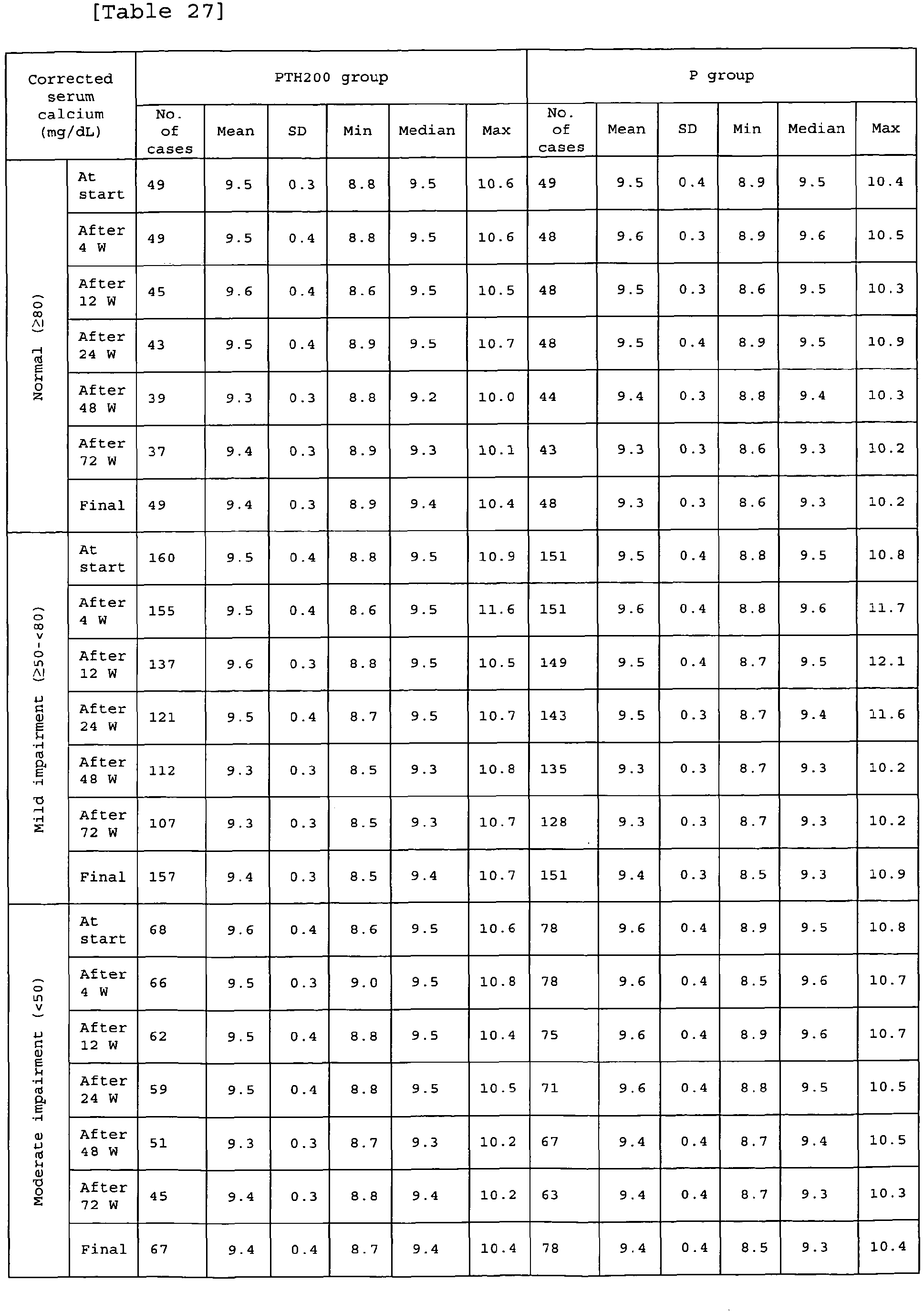

### (H-5) Safety of the test drug in each group (incidence of adverse events)

The incidence of adverse events was studied after administering the test drug to the a group of osteoporosis patients having normal renal function, a group of osteoporosis patients having mild renal dysfunction, and a group of osteoporosis patients having moderate renal dysfunction.

**[Table 28]**

| Normal (≥80) | Adverse events | | | | Total | 95% confidence interval of each group (exact) | | Fisher's exact test p value (2-way) | Point estimate of the difference | 95% confidence interval of the difference (asymptote) (with continuity correction) | |
|---|---|---|---|---|---|---|---|---|---|---|---|
| | Yes | | No | | | | | | | | |
| | No. of cases | (%) | No. of cases | (%) | | Lower limit | Upper limit | | | Lower limit | Upper limit |
| PTH200 group | 46 | 93.9 | 3 | 6.1 | 49 | 83.1 | 98.7 | 1.0000 | 2.0 | -10.2 | 14.3 |
| P group | 45 | 91.8 | 4 | 8.2 | 49 | 80.4 | 97.7 | | | | |

**[Table 29]**

| Mild impairment (≥50-<80) | Adverse events | | | | Total | 95% confidence interval of each group (exact) | | exact test p (value 2-way) | Fisher's Point estimate of the difference | 95% confidence interval of the difference (asymptote) (with continuity correction) | |
|---|---|---|---|---|---|---|---|---|---|---|---|
| | Yes | | No | | | | | | | | |
| | No. of cases | (%) | No. of cases | (%) | | Lower limit | Upper limit | | | Lower limit | Upper limit |
| PTH200 group | 147 | 91.9 | 13 | 8.1 | 160 | 86.5 | 95.6 | 0.6689 | -1.5 | -7.9 | 4.9 |
| P group | 141 | 93.4 | 10 | 6.6 | 151 | 88.2 | 96.8 | | | | |

**[Table 30]**

| Moderate impairment (<50) | Adverse events | | | | Total | 95% confidence interval of each group (exact) | | Fisher's exact test p value (2-way) | Point estimate of the difference | 95% confidence interval of the difference (asymptote) (with continuity correction) | |
|---|---|---|---|---|---|---|---|---|---|---|---|
| | Yes | | No | | | | | | | | |
| | No. of cases | (%) | No. of cases | (%) | | Lower limit | Upper limit | | | Lower limit | Upper limit |
| PTH200 group | 65 | 95.6 | 2 | 4.4 | 68 | 87.6 | 99.1 | 0.3385 | 4.6 | -4.8 | 13.9 |
| P group | 71 | 91.0 | 7 | 9.0 | 78 | 82.4 | 96.3 | | | | |

### (H-6) Safety of the test drug in each group (incidence of side effects)

The incidence by the test drug in each group was about twice that of the control as a result of administering the test drug to the a group of osteoporosis patients having normal renal function, a group of osteoporosis patients having mild renal dysfunction, and a group of osteoporosis patients having moderate renal dysfunction. In other words, the safety of the test drug as regards side effects was clarified to be equivalent in all groups.

**[Table 31]**

| Normal (≥80) | Side effects | | | | Total | 95% confidence interval of each group (exact) | | Fisher's exact test p value (2-way) | Point estimate of the difference | 95% confidence interval of the difference (asymptote) (with continuity correction) | |
|---|---|---|---|---|---|---|---|---|---|---|---|
| | Yes | | No | | | | | | | | |
| | No. of cases | (%) | No. of cases | (%) | | Lower limit | Upper limit | | | Lower limit | Upper limit |
| PTH200 group | 22 | 44.9 | 27 | 55.1 | 49 | 30.7 | 59.8 | 0.0318 | 22.4 | 2.2 | 42.7 |
| P group | 11 | 22.4 | 38 | 77.6 | 49 | 11.8 | 36.6 | | | | |

**[Table 32]**

| Mild impairment (≥50-<80) | Side effects | | | | Total | 95% confidence interval of each group (exact) | | Fisher's exact test p value (2-way) | Point estimate of the difference | 95% confidence interval of the difference (asymptote) (with continuity correction) | |
|---|---|---|---|---|---|---|---|---|---|---|---|
| | Yes | | No | | | | | | | | |
| | No. of cases | (%) | No. of cases | (%) | | Lower limit | Upper limit | | | Lower limit | Upper limit |
| PTH200 group | 72 | 45.0 | 88 | 55.0 | 160 | 37.1 | 22.7 | <.00001 | 29.1 | 18.8 | 39.4 |
| P group | 24 | 15.9 | 127 | 84.1 | 151 | 10.5 | 53.1 | | | | |

**[Table 33]**

| Moderate impairment (<50) | Side effects | | | | Total | 95% confidence interval of each group (exact) | | Fisher's exact test p value (2-way) | Point estimate of the difference | 95% confidence interval of the difference (asymptote) (with continuity correction) | |
|---|---|---|---|---|---|---|---|---|---|---|---|
| | Yes | | No | | | | | | | | |
| | No. of cases | (%) | No. of cases | (%) | | Lower limit | Upper limit | | | Lower limit | Upper limit |
| PTH200 group | 25 | 36.8 | 43 | 63.2 | 68 | 25.4 | 49.3 | 0.0251 | 17.5 | 1.7 | 33.3 |
| P group | 15 | 19.2 | 63 | 80.8 | 78 | 11.2 | 29.7 | | | | |

### (I) Effects of test drug administration on changes over time in the incidence of new vertebral fractures

The test drug group was listed as the "PTH200 group" and the control group as the "P group."

**[Table 34]**

| FAS | No. evaluated | No. discontinued | No. with fractures | Incidence of new vertebral fractures (%) | | | Difference between PTH200 group and P group after 72 W (%) | | | log rank test |
|---|---|---|---|---|---|---|---|---|---|---|
| | | | | | | | Difference | 90% confidence interval | | |
| | | | | After 24 W | After 48 W | After 72 W | | Lower limit | Upper limit | |
| PTH200 | 261 | 58 | 7 | 2.6 | 3.1 | 3.1 | 11.4 | 7.3 | 15.4 | <.0001 |
| P | 281 | 31 | 37 | 5.3 | 10.4 | 14.5 | | | | |

**[Table 35]**

| | New vertebral fractures | | | | | | | |
|---|---|---|---|---|---|---|---|---|
| | PTH200 group | | | | P group | | | |
| Time after administration | No. evaluated | No. with fractures | Incidence of fractures(%) | No. of fractures | No. evaluated | No. with fractures | Incidence of fractures(%) | No. of fractures |
| ≤ 24 W | 261 | 6 | 2.3 | 7 | 281 | 14 | 5.0 | 18 |
| > 24 W-≤ 48 W | 219 | 2 | 0.9 | 2 | 257 | 13 | 5.1 | 13 |
| > 48 W-≤ 72 W | 206 | 0 | 0 | 0 | 245 | 13 | 5.3 | 15 |

As shown in the above tables, the incidence of new vertebral fractures every six months was basically steady at approximately 5% in all divisions of the P group. In contrast to this, the incidence of each division dropped as the administration period lengthened in the PTH200 group, and no new vertebral fractures occurred after 48 weeks. The incidence of new vertebral fractures was also lower than that of the P group in all divisions consisting of within 24 weeks, 24 to 48 weeks, and 48 to 72 weeks, and the relative risk reduction (RRR) in comparison to the placebo increased as administration continued. Thus, weekly administration of 200 units of this drug inhibited the occurrence of new vertebral fractures from early on, and the risk of fractures had already dropped 53.9% versus the placebo after 24 weeks. The inhibitory effect of this drug on bone fractures also tended to intensify as administration continued.

The incidence of vertebral fractures (new + worsening) after 72 weeks by Kaplan-Meier estimation in the FAS of the fracture study was 3.5% in the PTH200 group and 16.3% in the P group. The incidence with 200 units of this drug was lower than in the placebo group (log rank test, p < 0.0001). Two hundred units of this drug also lowered the risk of vertebral fractures (new + worsening) 78.6% in comparison to the placebo after 72 weeks. When the six-month vertebral fracture (new + worsening) incidence was compared between groups, the incidence of the PTH200 group was lower than that of the P group in all divisions consisting of within 24 weeks, 24 to 48 weeks, and 48 to 72 weeks.

### (J) Effects of test drug administration on the urinary calcium and serum calcium of osteoporosis patients

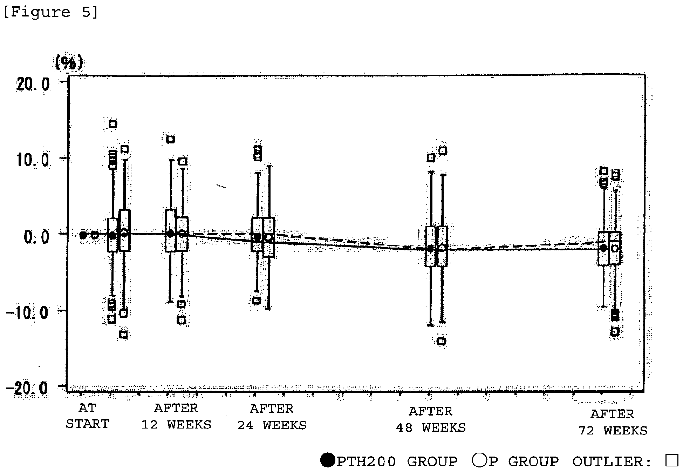

The test drug group was listed as the "PTH200 group" and the control group as the "P group." The results of studying the changes in the urinary calcium value and corrected serum calcium value when the test drug or control was administered to patients once a week for 72 weeks are shown (Figures 4 and 5) .

The average (and median) percentage change in the urinary calcium value was 3.2% (-14.7%) in the PTH200 group and 23.6% (1.6%) in the P group after 72 weeks in comparison to at the start. A tendency to decrease was seen in the PTH200 group in comparison to the P group.

The corrected serum calcium value fluctuated with a range of an average of 9.3 to 9.6 mg/dL in both groups. The corrected serum calcium value of the PTH200 group after administration had a minimum of 8.5 mg/dL (after 48 and 72 weeks) and a maximum of 11.6 mg/dL (after 4 weeks). In the P group, it had a minimum of 8.5 mg/dL (after 4 weeks) and a maximum of 12.1 mg/dL (after 12 weeks). No large fluctuations were found in either group.

No adverse effects of serum calcium elevation or depression were found in this study.

No manifestation of hypercalcemia or hypercalciuria was found in the PTH200 group in comparison to the P group in this study.

### INDUSTRIAL APPLICABILITY

The method for treating/preventing osteoporosis and inhibiting/preventing bone fractures disclosed herein is excellent in terms of both efficacy and safety, and the method for inhibiting bone fractures disclosed herein is highly safe. Both are groundbreaking medical techniques that contribute significantly to the treatment of osteoporosis and the like and the inhibition/prevention of bone fractures. The osteoporosis therapeutic/prophylactic agent and agent for the inhibition/prevention of bone fractures of the present invention are therefore extremely useful in the pharmaceutical industry for these purposes.

## Claims

1. An agent containing parathyroid hormone (PTH) as the active ingredient for use in the treatment or prevention of osteoporosis in a patient, wherein the PTH containing agent is to be administered once a week in a unit dose of 200 units over a duration exceeding 48 weeks, and wherein the patient satisfies all of the following conditions (1) through (3):
(1) age 65 years or older;
(2) prevalent bone fractures;
(3) bone density less than 80% of the young adult mean and/or a degree of bone atrophy of l or higher.

2. The agent for use according to claim 1, wherein the PTH is human PTH (1-34).

## Patentansprüche

1. Mittel, welches Parathyroidhormon (PTH) als aktiven Wirkstoff enthält, für die Verwendung bei der Behandlung oder Vorbeugung von Osteoporose in einem Patienten, wobei das PTH-enthaltende Mittel einmal wöchentlich in einer Dosiseinheit von 200 Einheiten für eine Dauer von mehr als 48 Wochen zu verabreichen ist, und wobei der Patient sämtliche der folgenden Bedingungen (1) bis (3) erfüllt:
(1) Alter 65 Jahre oder älter;
(2) prävalente Knochenfrakturen;
(3) Knochendichte weniger als 80% des Mittelwertes eines jungen Erwachsenen und/oder einen Grad der Knochenatrophie von l oder höher.

2. Das Mittel für die Verwendung gemäß Anspruch 1, wobei es sich bei dem PTH um menschliches PTH (1-34) handelt.

## Revendications

1. Agent contenant de l'hormone parathyroïdienne (PTH) comme ingrédient actif destiné à une utilisation dans le traitement ou la prévention de l'ostéoporose chez un patient, l'agent contenant la PTH devant être administré une fois par semaine sous une dose unitaire de 200 unités sur une durée excédant 48 semaines, et le patient satisfaisant toutes les conditions suivantes (1) à (3) :
(1) être âgé-e de 65 ans ou plus ;
(2) fractures courantes des os ;
(3) densité osseuse inférieure à 80 % de la moyenne chez l'adulte jeune et/ou degré d'atrophie osseuse de 1 ou supérieur.

2. Agent destiné à une utilisation selon la revendication 1, la PTH étant la PTH humaine (1-34).
